# EUROPEAN PATENT SPECIFICATION

(11) **EP 1 855 734 B1**
(45) Date of publication and mention of the grant of the patent: **12.02.2014**
(21) Application number: 06710014.9
(22) Date of filing: 06.03.2006
(51) Int. Cl.: A61L 27/46, A61L 27/56

(54) **BIOMATERIAL**
BIOMATERIAL
BIOMATERIAU

(30) Priority: 07.03.2005 GB 0504673
(43) Date of publication of application: 21.11.2007
(73) Proprietor: Cambridge Enterprise Limited, Trinity Lane Cambridge Cambridgeshire CB2 1TN (GB); MASSACHUSETTS INSTITUTE OF TECHNOLOGY, Cambridge, Massachusetts 02139 (US)
(72) Inventor: LYNN, Andrew K c/o Cambridge Entreprise Limited, Cambridge Cambridgeshire CB2 1QA (GB); BONFIELD, William c/o Cambridge Entreprise Limited, Cambridge Cambridgeshire CB2 1QA (GB); GIBSON, Lorna J, Massachusetts 02139-4307 (US); YANNAS, Ioannis V, Massachusetts 02139-4307 (US); HARLEY, Brendan A, Cambridge, Massachusetts 02139 (US)
(74) Representative: Murphy, Joeeta
(86) International application number: PCT/GB2006/000797
(87) International publication number: WO 2006/095154

(56) References cited:
- EP-A- 1 457 214
- EP-A- 1 500 405
- WO-A-03/092760
- WO-A-2004/041320
- WO-A-2005/051447
- WO-A1-2004/103422
- WO-A2-2006/092718

## Description

The present invention relates to the field of synthetic bone materials for biomedical applications and, in particular, to porous monolithic and porous layered scaffolds comprising collagen, calcium phosphate, and optionally a glycosaminoglycan for use in tissue engineering.

Natural bone is a biocomposite of collagen, non-collagenous organic phases including glycosaminoglycans, and calcium phosphate. Its complex hierarchical structure leads to exceptional mechanical properties including high stiffness, strength, and fracture toughness, which in turn enable bones to withstand the physiological stresses to which they are subjected on a daily basis. The challenge faced by researchers in the field is to make a synthetic material that has a composition and structure that will allow natural bone growth in and around the synthetic material in the human or animal body.

It has been observed that bone will bond directly to calcium phosphates in the human body (a property referred to as bioactivity) through a bone-like apatite layer formed in the body environment. Collagen and copolymers comprising collagen and other bioorganics such as glycosaminoglycans on the other hand, are known to be optimal substrates for the attachment and proliferation of numerous cell types, including those responsible for the production and maintenance of bone in the human body.

Hydroxyapatite is the calcium phosphate most commonly used as a constituent in bone substitute materials. It is, however, a relatively insoluble material when compared to other forms of calcium phosphate materials such as brushite, tricalcium phosphate and octacalcium phosphate. The relatively low solubility of apatite can be a disadvantage when producing a biomaterial as the rate of resorption of the material in the body is particularly slow.

Calcium phosphates such as hydroxyapatite are mechanically stiff materials. However, they are relatively brittle when compared to natural bone. Collagen is a mechanically tough material, but has relatively low stiffness when compared to natural bone. Materials comprising copolymers of collagen and glycosaminoglycans are both tougher and stiffer than collagen alone, but still have relatively low stiffness when compared to natural bone.

Previous attempts to produce a synthetic bone-substitute material having improved mechanical toughness over hydroxyapatite and improved stiffness over collagen and copolymers of collagen and glycosaminoglycans include combining collagen and apatite by mechanical mixing. Such a mechanical method is described in EP-A-0164 484.

Later developments include producing a bone-replacement material comprising hydroxyapatite, collagen and chondroitin-4-sulphate by the mechanical mixing of these components. This is described in EP-A-0214070. This document further describes dehydrothermic crosslinking of the chondroitin-4-sulphate to the collagen. Materials comprising apatite, collagen and chondroitin-4-sulphate have been found to have good biocompatibility. The mechanical mixing of the apatite with the collagen, and optionally chondroitin-4-sulphate, essentially forms collagen / chondroitin-4-sulphate-coated particles of apatite. It has been found that such a material, although biocompatible, produces limited in-growth of natural bone when in the human or animal body and no remodeling of the calcium phosphate phase of the synthetic material.

The repair of skeletal sites compromised by trauma, deformity or disease poses a special challenge to orthopaedic surgeons in that, unlike defects in skin, nerve and most other tissue types, skeletal defects encompass multiple, distinct tissue types (i.e. bone, cartilage, tendon and ligament), involve locations that undergo regular mechanical loading, and traverse interfaces between mineralised to unmineralised tissues (e.g. ligament insertion points, the "tidemark" at the bone/cartilage interface).

Existing clinical approaches address the repair of skeletal defects either with non-resorbable prosthetic implants, autologous or allogenous tissue grafts, chemical agents, cell transplantation or combinations of these methods. While these approaches have achieved some success for the treatment of single tissue types, cases where interfaces between mineralised and unmineralised tissue are involved, such as articular joint defects for example, result in healing that is, at best, incomplete. Furthermore, even the most successful of the existing treatments require either the harvest of tissue from a donor site and/or the suturing to bone, cartilage, ligament or tendon. The former procedure suffers from lack of donor sites and donor site morbidity, while the latter is difficult to implement and creates additional defects in the form of suture holes.

The terms composite scaffold and layered scaffold are synonymous, and refer to scaffolds comprising two or more layers, with the material composition of each layer differing substantially from the material composition of its adjacent layer or layers. The term single-layered scaffold or monolithic scaffold are synonymous, and refer to scaffolds comprising one layer only, with the material composition within each layer being largely homogeneous throughout.

A limited number of recent efforts have sought to develop tissue-engineering strategies that employ porous, layered scaffolds for the treatment of articular joint defects involving either cartilage alone or both bone and cartilage. These constructs seek to induce the regeneration of bone and cartilage concurrently, but using separate scaffolds for each (Niederauer et al., 2000; Schaefer et al., 2000; Gao et al., 2001; Gao et al., 2002; Schaefer et al., 2002; Sherwood et al., 2002; Hung et al., 2003; Hunziker and Driesang, 2003).

An additional feature of layered scaffolds is the potential they hold for achieving sutureless fixation via direct attachment of the bony layer to the subchondral bone plate. Provided the cartilaginous portion remains firmly attached to the bony portion, no additional fixation is required. Sutureless fixation may also enable the treatment of defects involving insertions points of tendon and ligament to bone.

Despite the promise of this new approach, two shortcomings can limit the effectiveness of the layered scaffolds reported to date. The first relates to the materials used for the respective layers of the scaffold. Resorbable synthetic polymers have been the only material used for the cartilaginous layer, and have often been a component of the osseous portion in many of these scaffolds as well. Although easy to fabricate, synthetic polymers are known to be less conducive to cell attachment and proliferation than natural polymers such as collagen, and can furthermore release high concentrations of acid as they degrade. Moreover, for applications where tendon or ligament repair is necessary, resorbable synthetic polymers, regardless of the manner in which they are crosslinked, have inadequate strength and stiffness to withstand even the reduced load applied during rehabilitation exercises.

The second shortcoming of conventional layered scaffolds relates to the interface between the respective layers. Natural articular joints and tendon/ligament insertion points are characterised by continuity of collagen fibrils between the mineralised and unmineralised regions. The resultant system of smooth transitions (soft interfaces) imparts an intrinsic mechanical stability to these sites, allowing them to withstand physiological loading without mechanical failure. In contrast, the majority of existing layered scaffolds contain hard interfaces, forming a distinct boundary between two dissimilar materials. Suturing (Schaefer et al., 2000), fibrin adhesive bonding (Gao et al., 2001) and other techniques (Gao et al., 2002; Hung et al., 2003) have been used to strengthen this interface. However, interfacial debonding has still been reported even in controlled animal models. These suturing and bonding methods are also delicate and poorly reproducible.

Previous work has developed means through which the parameters of freeze-drying protocols can be controlled to produce porous scaffolds of collagen and one or more glycosaminoglycans (GAGs) (Yannas et al., 1989; O'Brien et al., 2004; O'Brien et al., 2005; Loree et al 1989). These techniques allow scaffold features such as pore size and aspect ratio to be varied in a controlled manner, parameters known to have marked effects on the healing response at sites of trauma or injury. However, for treatment of injuries involving skeletal and musculoskeletal defects, it is necessary to develop technologies to produce porous scaffolds with material compositions and mechanical characteristics that closely match those of bone, as opposed to those of unmineralised collagen-GAG scaffolds.

WO 2004/041320 discloses a monolithic resorbable cross-linked apatite/collagen porous body and a process for making same. The process comprises a gelation step prior to freeze-drying which improves the rupture strength of the resulting porous body.

EP1500405 discloses porous and composite materials with large pore sizes, high porosities and mechanical strengths that are suitable for implants such as bone fillers. The materials are produced by freezing a complex containing a calcium salt, hydroxyapatite and collagen, at least a part of which is gelatinized, and then lyophilising the resultant.

WO 03/092760 discloses a structured composite for tissue engineering consisting of a mass of porous calcium phosphate granules bound by a biocompatible binder being collagen or hyaluronic acid. The process for making the material comprises mixing a solution or suspension of binder with calcium phosphate granules, deep freezing and thawing, and freeze drying of the mixture.

The present invention seeks to address at least some of the problems associated with the prior art.

The present invention discloses:

A process for the preparation of a composite biomaterial suitable for repairing interfaces between tissues comprising collagen and a calcium phosphate material, or collagen, a calcium phosphate material and a glycosaminoglycan, the process comprising:
(a) providing a first slurry composition comprising a liquid carrier, collagen, and a calcium phosphate material; and providing a second slurry composition comprising a liquid carrier and collagen; or
   providing a first slurry composition comprising a liquid carrier, collagen, and a calcium phosphate material, and providing a second slurry composition comprising a liquid carrier, collagen, and a calcium phosphate material; or
   providing a first slurry composition comprising a liquid carrier, collagen, and a calcium phosphate material; and providing a second slurry composition comprising a liquid carrier, collagen, a calcium phosphate material, and at glycosaminoglycan; or
   providing a first slurry composition comprising a liquid carrier, collagen, a calcium phosphate material, and a glycosaminoglycan; and providing a second slurry composition comprising a liquid carrier and collagen; or
   providing a first slurry composition comprising a liquid carrier, collagen, a calcium phosphate material, and a glycosaminoglycan; and providing a second slurry composition comprising a liquid carrier, collage, and a calcium phosphate material; or
   providing a first slurry composition comprising a liquid carrier, collagen, a calcium phosphate material, and a glycosaminoglycan; and providing a second slurry composition comprising a liquid carrier, collagen, a calcium phosphate material, and a glycosaminoglycan;
(b) providing a mould for the slurry;
(c) depositing the first and second slurries in the mould;
(d) cooling the slurry deposited in the mould to a temperature at which the liquid carrier transforms into a plurality of solid crystals or particles;
(e) removing at least some of the plurality of solid crystals or particles by sublimation and/or evaporation to leave a porous composite material comprising collagen, a calcium phosphate material, and optionally a glycosaminoglycan; and
(f) removing the material from the mould.

The term biomaterial as used herein means a material that is biocompatible with a human or animal body.

The term slurry as used herein encompasses slurries, solutions, suspensions, colloids and dispersions.

The term collagen as used herein encompasses recombinant human (rh) collagen.

The inorganic material comprises a calcium phosphate material, the organic material comprises collagen and optionally a glycosaminoglycan. This results in a porous composite material comprising the calcium phosphate material and collagen and optionally a glycosaminoglycan. Preferably, the first slurry comprises a co-precipitate of collagen and the calcium phosphate material. More preferably, the first slurry comprises a triple co-precipitate of collagen, a calcium phosphate material and a glycosaminoglycan.

Alternatively, the first slurry may simply comprise a mechanical mixture of collagen and the calcium phosphate material and optionally the glycosaminoglycan. This may be produced by a conventional technique such as described in, for example, EP-A-0164 484 and EP-A-0214070. While a mechanical mixture may be used to form the slurry, a co-precipitate of collagen and the calcium phosphate material or a triple co-precipitate of collagen, the calcium phosphate material and a glycosaminoglycan are preferred.

The calcium phosphate material may be selected, for example, from one or more of brushite, octacalcium phosphate and/or apatite. The calcium phosphate material preferably comprises brushite.

The pH of the slurry is preferably from 2.5 to 6.5, more preferably from 2.5 to 5.5, still more preferably from 3.0 to 4.5, and still more preferably from 3.8 to 4.2.

The slurry composition may comprise one or more glycosaminoglycans. The slurry composition may comprise one or more calcium phosphate materials.

The presence of other species (e.g. silver, silicon, silica, table salt, sugar, etc) in the precursor slurry is not precluded.

At least some of the plurality of solid crystals or particles may be removed by sublimation and/or evaporation to leave a porous composite material comprising collagen, a calcium phosphate material, and optionally a glycosaminoglycan. The preferred method is sublimation.

Steps (d) and (e) may be effected by a freeze-drying technique. If the liquid carrier is water, the sublimation step comprises reducing the pressure in the environment around the mould and frozen slurry to below the triple point of the water/ice/water vapour system, followed by elevation of the temperature to greater than the temperature of the solid-vapor transition temperature at the achieved vacuum pressure. The ice in the product is directly converted into vapor via sublimation as long as the ambient partial liquid vapor pressure is lower than the partial pressure of the frozen liquid at its current temperature. The temperature is typically elevated to at or above 0°C. This step is performed to remove the ice crystals from the frozen slurry via sublimation.

The freeze-drying parameters may be adjusted to control pore size and aspect ratio as desired. In general, slower cooling rates and higher final freezing temperatures (for example, cooling at approximately 0.25°C per minute to a temperature of about -10°C) favour large pores with higher aspect ratios, while faster cooling rates and lower final freezing temperatures (for example, cooling at approximately 2.5°C per minute to a temperature of about -40°C) favours the formation' of small equiaxed pores.

The term "mould" as used herein is intended to encompass any mould, container or substrate capable of shaping, holding or supporting the slurry composition. Thus, the mould in its simplest form could simply comprise a supporting surface. The mould may be any desired shape, and may be fabricated from any suitable material including polymers (such as polysulphone, polypropy-ene, polyethylene), metals (such as stainless steel, titanium, cobalt chrome), ceramics (such as alumina, zirconia), glass ceramics, and glasses (such as borosilicate glass) .

The applicant's earlier application, WO 2005/051447, filed 28:October 2004, describes a triple co-precipitate of collagen, brushite and a glycosaminoglycan and a process for its preparation.

The process described in WO 2005/051447 involves: providing an acidic aqueous solution comprising collagen, a calcium source and a phosphorous source and a glycosaminoglycan; and precipitating the collagen, the brushite and the glycosaminoglycan together from the aqueous solution to form a triple co-precipitate.

The term co-precipitate means precipitation of the two or three compounds where the compounds have been precipitated at substantially the same time from the same solution/dispersion. It is to be distinguished from a material formed from the mechanical mixing of the components, particularly where these components have been precipitated separately, for instance in different solutions. The microstructure of a co-precipitate is substantially different from a material formed from the mechanical mixing of its components.

In the process for preparing the co-precipitate, the calcium source is preferably selected from one or more of calcium nitrate, calcium acetate, calcium chloride, calcium carbonate, calcium alkoxide, calcium hydroxide, calcium silicate, calcium sulphate, calcium gluconate and the calcium salt of heparin. A calcium salt of heparin may be derived from the porcine intestinal mucosa. Suitable calcium salts are commercially available, for example, from Sigma-Aldrich Inc. The phosphorus source is preferably selected from one or more of ammonium-dihydrogen phosphate, diammonium hydrogen phosphate, phosphoric acid, disodium hydrogen orthophosphate 2-hydrate (Na₂HPO₄.2H₂O, sometimes termed GPR Sorensen's salt) and trimethyl phosphate, alkali metal salts (eg Na or K) of phosphate, alkaline earth salts (eg Mg or Ca) of phosphate.

Glycosaminoglycans are a family of macromolecules containing long unbranched polysaccharides containing a repeating disaccharide unit. Preferably, the glycosaminoglycan is selected from one or more of chondroitin sulphate, dermatin sulphate, heparin, heparin sulphate, keratin sulphate and hyaluronic acid. Chondroitin sulphate may be chondroitin-4-sulphate or chondroitin-6-sulphate, both of which are commercially available, for example, from Sigma-Aldrich Inc. The chondroitin-6-sulphate may be derived from shark cartilage. Hyaluronic acid may be derived from human umbilical chord. Heparin may be derived from porcine intestinal mucosa.

The collagen may be soluble or insoluble and may be derived from any tissue in any animal and may be extracted using any number of conventional techniques.

Precipitation may be effected by combining the collagen, the calcium source, the phosphorous source and the glycosaminoglycan in an acidic aqueous solution and either allowing the solution to stand until precipitation occurs, agitating the solution, titration using basic titrants such as ammonia, addition of a nucleating agent such as prefabricated brushite, varying the rate of addition of the calcium source, or any combination of these or numerous other techniques known in the art.

It will be appreciated that other components may be present in the slurry. For example, growth factors, genes, drugs or other biologically active species may optionally be added, alone or in combination, to the slurry.

In a preferred embodiment, the process according to the present invention advantageously further comprises: providing a second slurry composition comprising a liquid carrier and an organic material and optionally an inorganic material; and
prior to said cooling step, depositing said second slurry composition in the mould either before or after said first slurry composition has been deposited.

As before, the organic material will typically comprise one or more of collagen (including recombinant human (rh) collagen), a glycosaminoglycan, albumin, hyaluronan, chitosan, and synthetic polypeptides comprising a portion of the polypeptide sequence of collagen.

The second slurry composition may comprise an inorganic material such as, for example, a calcium phosphate material.

Preferably, the second slurry composition comprises a liquid carrier, collagen, optionally a calcium phosphate material, and optionally a glycosaminoglycan. In this embodiment, the second slurry composition preferably comprises a co-precipitate of collagen and a glycosaminoglycan, or a co-precipitate of collagen and a calcium phosphate material, or a triple co-precipitate of collagen, a glycosaminoglycan and a calcium phosphate material. Co-precipitation has already been discussed in relation to the preparation of the first slurry.

Alternatively, the second slurry may simply comprise a mechanical mixture of collagen and optionally one or both of a calcium phosphate material and a glycosaminoglycan. Mechanical mixtures have already been discussed in relation to the preparation of the first slurry.

If present, the calcium phosphate material in.the second slurry may be selected from one or more of brushite, octacalcium phosphate and/or apatite.

The first and second slurry compositions will typically be deposited as first and second layers in the mould. For example, the first slurry is deposited in the mould, followed by the second slurry. The mould contents may then be subjected to steps (d), (e) and (f). Accordingly, the process may be used to form a multi-layered material, at least one layer of which preferably comprises a porous composite material comprising collagen, a calcium phosphate material, and optionally a glycosaminoglycan. The layer resulting from the second slurry composition may be a porous or a non-porous layer. If a porous layer is desired, then the pores can be created by sublimation and/or evaporation of a plurality of solid crystals or particles formed in the second slurry. This technique has been already discussed in relation to the first slurry and preferably comprises a freeze drying technique.

The process is carried out in the liquid phase and this is conducive to diffusion between the first slurry layer and the second slurry layer.

The layers may be deposited in any manner of layering orders or geometries. The layers may, for example, be situated vertically (i.e. one on top of the other), horizontally (i.e. one beside the other), and/or radially (one spherical layer on top of the next).

The casting process according to the present invention enables the fabrication of porous monolithic and porous layered scaffolds for use in tissue engineering.

After the first and second slurry compositions have been deposited in the mould, the contents of the mould are preferably left to rest for up to 24 hours before the cooling step. This is advantageous because it allows inter-diffusion of the various slurry constituents between adjacent layers. This results in an improvement in inter-layer bond strength.

The liquid carrier in the first slurry preferably comprises water. The liquid carrier in the second slurry also preferably comprises water.

It will be appreciated that further slurry layers may be deposited in the mould prior to said cooling step, either before or after said first and/or second slurry composition(s) has/have been deposited.

The temperature of the slurry deposited in the mould prior to the cooling step will generally have an effect on the viscosity of the slurry. If the temperature is too high, then this may result in slurries of excessively low viscosity, which may result in complete (and therefore undesirable) intermixing of the first and second layers once the second slurry is deposited. It should also be noted that too high a temperature may result in denaturation of the collagen. On the other hand, too low a temperature may result in slurries with viscosities too high to allow efficient spreading, smoothing or shaping, and may risk the premature formation of ice crystals. Accordingly, the inventors have found that the temperature of the first slurry deposited in the mould prior to the cooling step is preferably in the range of from 2 to 40°C, more preferably from 4 to 37°C, still more preferably from 20 to 37°C. If multiple layered slurry compositions are used, then these ranges are also applicable to the additional slurries.

The step of cooling the first slurry deposited in the mould is preferably carried out to a temperature of ≤ 0°C. More preferably, the step of cooling is carried out to a temperature in the range of from -100 to 0°C, preferably from -80 to -10°C, more preferably from -40 to -20°C. If multiple layered slurry compositions are used, then these ranges are also applicable to the additional slurries.

The step of cooling the first slurry deposited in the mould is preferably carried out at a cooling rate of 0.02 - 10°C/min, more preferably from 0.02 - 6.0°C/min, still more preferably from 0.2 - 2.7°C/min. If multiple layered slurry compositions are used, then these ranges are also applicable to the additional slurries.

In general, slower cooling rates and higher final freezing temperatures (for example, cooling at 0.25°C per minute to a temperature of -10°C) favour large pores with higher aspect ratios, while faster cooling rates and lower final freezing temperatures (for example, cooling at 2.5°C per minute to a temperature of -40°C) favours the formation of small equiaxed pores.

The step of cooling the slurry deposited in the mould is preferably carried out at a pressure of from 1 - 200 kPa, more preferably from 50 - 150 kPa, still more preferably from 50 - 101.3 kPa. If multiple layered slurry compositions are used, then these ranges are also applicable to the additional slurries. The inventors have found that pressures below 50 kPa can result in the formation of bubbles within the slurry, while pressures greater than 200 kPa may induce excessive mixing of adjacent layers.

The thickness of the first slurry deposited in the mould is preferably from 0.1 - 500 mm, more preferably from 0.5 - 20 mm, still more preferably from 1.0 - 10 mm. If multiple layered slurry compositions are used, then these ranges are also applicable to the additional slurries. Layers in excess of 500 mm in thickness can be difficult to solidify completely, while layers less than 0.1 mm thick can freeze almost instantaneously, making it difficult to control accurately the progression of ice crystal nucleation and growth.

The viscosity of the first slurry prior to it being deposited in the mould is preferably from 0.1 - 50 Pa·s, more preferably from 0.1 - 10 Pa·s, still more preferably from 0.5 - 5 Pa·s. If multiple layered slurry compositions are used, then these ranges are also applicable to the additional slurries. Slurries with overly high viscosity can be difficult to spread, smooth and shape, while those with excessively low viscosity may result in complete (and therefore undesirable) intermixing of the first and second layers once the second slurry is deposited.

The step of removing at least some of the solid crystals or particles in the first slurry by sublimation is preferably carried out at a pressure of from 0 - 0.08 kPa, more preferably from 0.0025 - 0.08 kPa, still more preferably from 0.0025 - 0.04 kPa. If multiple layered slurry compositions are used, then these ranges are also applicable to the additional slurries. Pressures above that of the triple point of water (approximately 0.08 kPa) can risk the occurrence of melting instead of sublimation, while excessively low pressures are difficult to achieve, and unnecessary for encouraging sublimation.

With regard to the step of removing at least some of the solid crystals or particles in the first slurry by sublimation, if the duration of sublimation is too short, residual water and solvents can cause redissolution of the scaffold walls, thereby compromising the pore architecture. Accordingly, the inventors have found that this step is preferably carried out for up to 96 hours, more preferably from 12 - 72 hours, still more preferably from 24 - 36 hours. If multiple layered slurry compositions are used, then these ranges are also applicable to the additional slurries.

The step of removing at least some of the solid crystals or particles in the first slurry by sublimation is preferably carried out at a temperature of from -10 - 60°C, more preferably from 0 - 40°C, still more preferably from 20 - 37°C, still more preferably from 25 - 37°C. If multiple layered slurry compositions are used, then these ranges are also applicable to the additional slurries. If the temperature during sublimation is too low, the time required until sublimation is complete can become excessively long, while excessively high temperatures (i.e. above 40°C) can risk denaturation of the collagen.

If the material comprises collagen and a glycosaminoglycan, then the process according to the present invention may further comprise the step of cross-linking the collagen and the glycosaminoglycan in the porous composite biomaterial. Cross-linking-will typically take place after the material has been removed from the mould following sublimation. Crosslinking may be effected by subjecting the co-precipitate to one or more of gamma radiation, ultraviolet radiation, a dehyrdothermal treatment, non-enzymatic glycation with a simple sugar such as glucose, mannose, ribose and sucrose, contacting the triple co-precipitate with one or more of glutaraldehyde, carbodiimide (eg ethyl dimethylaminopropyl carbodiimide) and/or nordihydroguariaretic acid, or any combination of these methods. These methods are conventional in the art.

If the material comprises calcium phosphate, then the process according to the present invention may further comprise the step of converting at least some of the calcium phosphate material in the porous composite biomaterial to another calcium phosphate phase. For example, the process may comprise the step of converting at least some of the brushite in the porous composite biomaterial to octacalcium phosphate and/or apatite. The conversion of the brushite to octacalcium phosphate and/or apatite is preferably effected by hydrolysation. Phase conversion will typically take place after the material has been removed from the mould (and optionally cross-linked).

Apatite is a class of minerals comprising calcium and phosphate and has the general formula: Ca₅(PO₄)₃(X), wherein X may be an ion that is typically OH⁻, F⁻ and Cl⁻, as well as other ions known to those skilled in the art. The term apatite also includes substituted apatites such as silicon-substituted apatites. The term apatite includes hydroxyapatite, which is a specific example of an apatite. The hydroxyapatite may also be substituted with other species such as, for example, silicon.

As mentioned above, further slurry layers may be deposited in the mould prior to said cooling step, either before or after said first and/or second slurry composition(s) has/have been deposited. The further slurry layers will also typically comprise, for example, a liquid carrier, collagen, optionally a calcium phosphate material, and optionally a glycosaminoglycan. The contents of the mould are preferably left to rest for up to 24 hours before the cooling step so as to allow inter-diffusion of the various slurry constituents between adjacent layers.

Accordingly, the present invention provides a process for the preparation of a composite biomaterial comprising one, two, or more layers. At least one of the layers preferably comprises a porous biocomposite of collagen, a calcium phosphate material, and also preferably a glycosaminoglycan. All of the layers preferably contain collagen.

The composite biomaterial according to the present invention may be used to fabricate, for example, a porous monolithic scaffold, or a multi-layered scaffold in which at least one layer is porous. The composite biomaterial according to the present invention is advantageously used as a tissue regeneration scaffold for musculoskeletal and dental applications.

The process according to the present invention preferably involves incorporating collagen as an organic constituent in the first and second layers (collagen is preferably the major organic constituent in the first and second layers). If additional layers are present, then the process preferably involves incorporating collagen as an organic constituent in one or more of these further layers (collagen is also preferably the major organic constituent in the one or more further layers). The process involves fabricating all layers, and thus the interfaces between them, simultaneously in the liquid phase. This results in the creation of a strong interface between the layers through inter-diffusion. The term inter-diffusion refers to mixing that occurs as a result of molecular diffusion or Brownian motion when two slurries of differing composition are placed in integral contact.

In a second aspect, the present invention provides a synthetic composite biomaterial, wherein at least part of the biomaterial is formed from a porous co-precipitate comprising a calcium phosphate material and one or more of collagen (including recombinant human (rh) collagen), a glycosaminoglycan, albumin, hyaluronan, chitosan or a synthetic polypeptides comprising a portion of the polypeptide sequence of collagen, wherein the macropore size range (pore diameter) is preferably from 1 - 1000 microns, more preferably from 200 - 600 microns. The material preferably comprises collagen. The calcium phosphate material is preferably selected from one or more of brushite, octacalcium phosphate and/or apatite. The porous material preferably comprises a co-precipitate of the collagen and the calcium phosphate material. This has already been described in relation to the first aspect of the invention.

The term porous as used herein means that the material may contain macropores and/or micropores. Macroporosity typically refers to features associated with pores on the scale of greater than approximately 10 microns. Microporosity typically refers to features associated with pores on the scale of less than approximately 10 microns.
It will be appreciated that there can be any combination of open and closed cells within the material. For example, the material will generally contain both macropores and micropores. The macroporosity is generally open-celled, although there may be a closed cell component.

The macropore size range (pore diameter) in the porous material according to the second aspect of the present invention is typically from 1 to 1200 microns, preferably from 10 to 1000 microns, more preferably from 100 to 800 microns, still more preferably from 200 to 600 microns.

The mean aspect ratio range in the porous material according to the second aspect of the present invention is preferably from 1 to 50, more preferably from 1 to 10, and most preferably approximately 1.

The pore size distribution (the standard deviation of the mean pore diameter) in the porous material according to the second aspect of the present invention is preferably from 1 to 800 microns, more preferably from 10 to 400 microns, and still more preferably from 20 to 200 microns.

The porosity in the porous material according to the second aspect of the present invention is preferably from 50 to 99.99 vol%, and more preferably from 70 to 98 vol%.

The percentage of open-cell porosity (measured as a percentage of the total number of pores both open- and closed-cell) in the porous material according to the second aspect of the present invention is preferably from 1 to 100%, more preferably from 20 to 100%, and still more preferably from 90 to 100%.

In a third aspect, the present invention provides a synthetic composite biomaterial, wherein at least part of the biomaterial is formed from a porous material comprising a calcium phosphate material and two or more of collagen (including recombinant human (rh) collagen), a glycosaminoglycan, albumin, hyaluronan, chitosan and a synthetic polypeptides comprising a portion of the polypeptide sequence of collagen. The material preferably comprises collagen and a glycosaminoglycan. The calcium phosphate material is preferably selected from one or more of brushite, octacalcium phosphate and/or apatite. The porous material preferably comprises a triple co-precipitate of collagen, a glycosaminoglycan and the calcium phosphate material. This has already been described in relation to the first aspect of the invention. The macropore size range (pore diameter) in the porous material according to the second aspect of the present invention is also applicable to the third aspect. The same is true for the mean aspect ratio range, the pore size distribution, the porosity and the percentage of open-cell porosity.

In a fourth aspect, the present invention provides a synthetic composite biomaterial comprising:
a first layer formed of a composite biomaterial according to the second or third aspect of the present invention; and
a second layer joined to the first layer and formed of a material comprising collagen, or a co-precipitate of collagen and a glycosaminoglycan, or a co-precipitate of collagen and a calcium phosphate material, or a triple co-precipitate of collagen, a glycosaminoglycan and a calcium phosphate material. The calcium phosphate material is preferably selected from one or more of brushite, octacalcium phosphate and/or apatite.

The first and second layers are preferably integrally formed. Advantageously, this may be achieved by a process involving liquid phase co-synthesis. This encompasses any process in which adjacent layers, either dense or porous, of a material comprising multiple layers are formed by placing the slurries comprising the precursors to each layer in integral contact with each other before removal of the liquid carrier or carriers from said slurries, and in which removal of said liquid carrier or carriers from all layers is preferably performed at substantially the same time. Placing the precursor slurries in integral contact before removal of the liquid carrier (i.e. while still in the liquid phase) allows interdiffusion to occur between adjacent slurries. This results in a zone of interdiffusion at the interface between adjacent layers of the resulting material, within which the material composition is intermediate to the material compositions of the adjacent layers. The existence of a zone of interdiffusion can impart mechanical strength and stability to the interface between adjacent layers. Accordingly, the first and second layers are preferably joined to one another through an inter-diffusion layer.

Alternatively, the first and second layers may be joined to one another through an inter-layer. The term inter-layer refers to any layer deposited independently between two other layers for the purpose of improving inter-layer bond strength or blocking the passage of cells, molecules or fluids between adjacent layers of the resulting scaffold, and may, for example, contain collagen, glycosaminoglycans, fibrin, anti-angiogenic drugs (e.g. suramin), growth factors, genes or any other constituents. An inter-layer is distinguished from an inter-diffusion layer by the fact that an inter-layer is deposited separately as a slurry whose composition is distinct from the composition of its adjacent layers, while an inter-diffusion layer is formed exclusively as a result of inter-diffusion between adjacent layers.

The first layer is porous. The second layer is also preferably porous, although it can be non-porous or substantially non-porous layer if desired.

The macropore size range (pore diameter) in the porous material according to the second aspect of the present invention is also applicable to the first and/or second layers in the embodiment according to the fourth aspect. The same is true for the mean aspect ratio range, the pore size distribution, the porosity and the percentage of open-cell porosity.

In any of the second, third and fourth aspects, the biomaterial may comprise one or more further layers joined to the first and/or second layers, each of said further layers preferably being formed of a material comprising collagen, or a co-precipitate of collagen and a glycosaminoglycan, or a co-precipitate of collagen and a calcium phosphate material, or a triple co-precipitate of collagen, a glycosaminoglycan, and a calcium phosphate material. The calcium phosphate material is preferably selected from one or more of brushite, octacalcium phosphate and/or apatite. The first and second layers and said one or more further layers are preferably integrally formed, and adjacent layers are preferably joined to one another through an inter-diffusion layer, which is typically formed by liquid phase co-synthesis. Generally, at least one of said further layers will be porous. Again, the macropore size range (pore diameter) in the porous material according to the second aspect of the present invention is also applicable to one or more of these further layers. The same is true for the mean aspect ratio range, the pore size distribution, the porosity and the percentage of open-cell porosity.

Differences in pore sizes between adjacent layers may vary from almost negligible to as great as +/- 1000 microns.

Unless otherwise stated, the following description is applicable to any aspect of the present invention.

If the material comprises collagen and a glycosaminoglycan, then the collagen and the glycosaminoglycan may be crosslinked.

The collagen is preferably present in the material in an amount of from 1 to 99 wt%, preferably from 5 to 90 wt%, more preferably from 15 to 60 wt%.

The glycosaminoglycan is preferably present in the material in an amount of from 0.01 to 20 wt%, more preferably from 1 to 12 wt%, still more preferably from 1 to 5.5 wt%.

If the material comprises brushite, then the ratio of collagen to brushite is preferably from 10:1 to 1:100 by weight, more preferably from 5:1 to 1:20 by weight.

If the material comprises octacalcium phosphate, then the ratio of collagen to octacalcium phosphate is preferably 10:1 to 1:100 by weight, more preferably from 5:1 to 1:20 by weight.

The ratio of collagen to the glycosaminoglycan is preferably from 8:1 to 30:1 by weight.

The biomaterial according to the present invention may be used as a substitute bone or dental material. Accordingly, the present invention provides a synthetic bone material, bone implant, bone graft, bone substitute, bone scaffold, filler, coating or cement comprising a biomaterial as herein described.

The biomaterial is advantageously provided in the form of a multi-layered scaffold. In particular, the present invention provides tissue regeneration scaffolds for musculoskeletal and dental applications. Multilayer (i.e. two or more layers) scaffolds according to the present invention may find application in, for example, bone/cartilage interfaces (eg articular joints), bone/tendon interfaces (eg tendon insertion points), bone/ligament interfaces (eg ligament insertion points), and tooth/ligament interfaces (eg tooth/periodontal ligament juncture).

Although the present invention is primarily concerned with scaffolds for tissue engineering applications, the material according to the present invention may be used to fabricate implants that persist in the body for quite some time. For example, a semi-permanent implant may be necessary for tendon and ligament applications.

The present invention further provides a porous composite biomaterial obtainable by a process as herein described.

### Synthesis Method

The present invention will now be described further by way of example. The preferred method of synthesis comprises a sequence of steps, which can be applied in whole or in part, to produce porous scaffolds having one or more layers at least one of which preferably comprises a triple co-precipitate of collagen, a glycosaminoglycan and a calcium phosphate material.

### Step 0: Slurry Preparation

The preparation of mineralised collagen/GAG/brushite slurry or slurries may be achieved using the method outlined in the applicant's earlier patent application, WO 2005/051447, filed 28 October 2004.

The preparation of unmineralised collagen/GAG slurry or slurries may be achieved using a method as outlined in Yannas et al., 1989; O'Brien et al., 2004; O'Brien et al., 2005); Loree et al., (1989).

Growth factors, genes, drugs or other biologically active species may optionally be added, alone or in combination, to the slurry via mechanical mixing at this stage to facilitate their incorporation into the scaffold. In the case of scaffolds with more than one layer, the biologically active species incorporated into one layer need not be the same as the species incorporated into the next.

### Step I: Casting

step I-a: Casting of 1st layer
Step I-b: Casting of 2nd layer
Step I-c: Casting of 3rd layer
Step I-n: Casting of nth layer

The casting step(s) involve the successive deposition of a slurry or slurries, in solution, suspension, colloid, or dispersion form, where water comprises the major diluent, into a mould, in which at least one of the slurries comprises a triple co-precipitate of collagen, one or more glycosaminoglycans and the calcium phosphate brushite, and all slurries contain collagen

The mould may' be any desired shape, and may be fabricated of any of a number of materials including polymers (such as polysulphone, polypropylene, polyethylene), metals (such as stainless steel, titanium, cobalt chrome) or ceramics (such as alumina, zirconia), glass ceramics, or glasses (such as borosilicate glass).

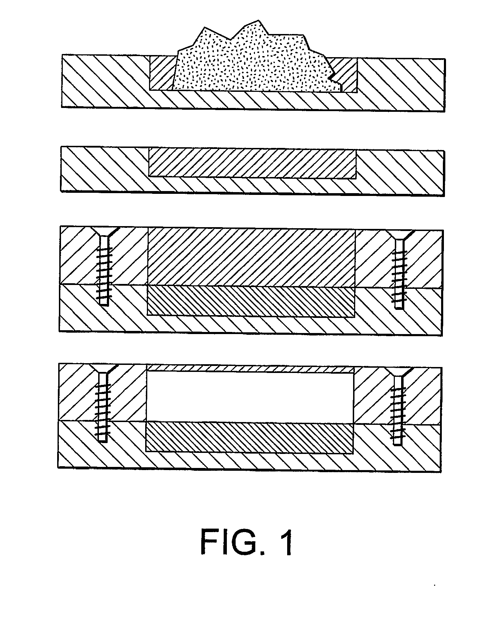

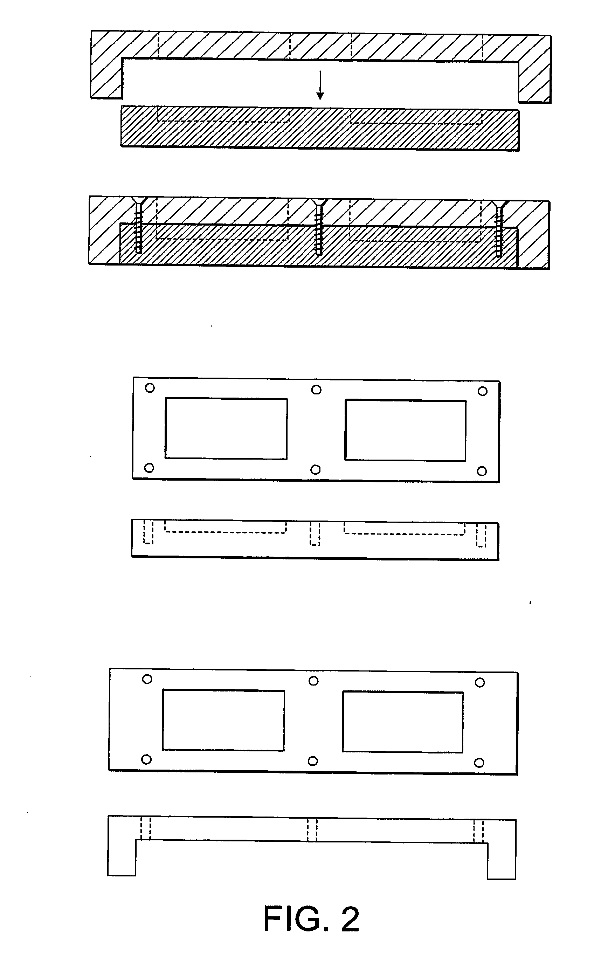

The mould may be constructed specifically to facilitate layering. Examples of suitable designs are shown in Figures 1 and 2.

The layers may, for example, be situated vertically (i.e. one on top of the other), horizontally (i.e. one beside the other), and/or radially (one spherical layer on top of the next).

In the event that the scaffold comprises two layers, at least one of the layers to be cast comprises a slurry of a co-precipitate comprising collagen, a calcium phosphate material, which is preferably brushite, and optionally a glycosaminoglycan. Preferably, the slurry comprises a triple co-precipitate comprising collagen, brushite, and a glycosaminoglycan. The preferred thickness of this layer is specified in the appropriate section of Table 1. The other layer comprises a slurry comprising collagen, optionally a calcium phosphate material, and optionally a glycosaminoglycan. This slurry composition preferably comprises a co-precipitate of collagen and a glycosaminoglycan, a co-precipitate of collagen and a calcium phosphate material such as brushite, or a triple co-precipitate of collagen, a glycosaminoglycan and a calcium phosphate material, which is preferably brushite.

Further layers may be included as desired and these further layers are preferably formed from a slurry comprising collagen, optionally a calcium phosphate material, and optionally a glycosaminoglycan. The further slurry compositions preferably comprise a co-precipitate of collagen and a glycosaminoglycan, a co-precipitate of collagen and a calcium phosphate material such as brushite, or a triple co-precipitate of collagen, a glycosaminoglycan and a calcium phosphate material, which is preferably brushite.

The composition of the slurries in each subsequent layer may be identical, vary slightly, or vary significantly, provided that collagen and preferably also a glycosaminoglycan are present in each layer, and that at least one of the layers also contains a calcium phosphate material such as, for example, brushite.

### Step II: Inter-diffusion

The co-diffusion step involves allowing the respective layers of the cast, layered slurry to inter-diffuse. This step is performed for the purpose of allowing inter-diffusion of slurry constituents between adjacent layers, thereby increasing the inter-layer bond strength after solidification and sublimation. Preferred conditions for the inter-diffusion step are listed in the appropriate section of Table 2.

### Step III: Controlled Cooling

The controlled cooling step involves placing the mould containing the slurry in an environment, which is then cooled at a controlled rate to a final temperature less than 0°C. This step is performed to initiate and control the rate of ice crystal nucleation and growth within the slurry. Ice crystals are then subsequently removed by sublimation leaving a porous scaffold. The architecture of the ice crystal network will determine the ultimate pore structure of the scaffold. The preferred parameters for cooling are listed in Table 3.

### Step IV: Annealing

The annealing step involves allowing the slurry to remain at the final temperature of the controlled cooling step for a designated amount of time. This step is performed to ensure that the slurry freezes completely or substantially completely. The preferred parameters for annealing are listed in Table 4.

### Step_V: Sublimation

The sublimation step comprises reducing, while the frozen slurry is maintained at roughly the final temperature of the controlled cooling and annealing steps, the pressure in the environment around the mould and frozen slurry to below the triple point of the water/ice/water vapour system, followed by elevation of the temperature to greater than the temperature of the solid-vapor transition temperature at the achieved vacuum pressure (typically ≥ 0°C). This step is performed to remove the ice crystals from the frozen slurry via sublimation. The advantage of sublimation over evaporation as a means of water removal is that it leaves a network of empty space (i.e. pores) that mimics precisely the architecture of the previously existing network of ice crystals. If the ice is allowed to melt, the ice crystal network loses its shape, and the architecture of the resulting pore network is compromised. Preferred parameters for the sublimation step are shown in Table 5.

### Step V + I: Crosslinking

If desired, the process may also involve a crosslinking step to crosslink the collagen and the glycosaminoglycan. This is described in the applicant's earlier patent application, WO 2005/051447, filed 28 October 2004.

### Examples

### Example I: Single-Layer Scaffold of Collagen/GAG/CaP

### (Reference Example)

### Materials

Collagen: Type I, microfibrillar collagen from bovine tendon, Integra Life Sciences Plainsboro, NJ, USA
GAG: Chondroitin-6-sulphate from shark cartilage, sodium salt, Sigma-Aldrich Inc (St. Louis, MO, USA)
Calcium Sources: (i) Calcium hydroxide (Ca(OH)₂), Sigma-Aldrich Inc (St. Louis, MO, USA); (ii) Calcium nitrate (Ca(NO₃)₂.4H₂O), Sigma-Aldrich Inc (St. Louis, MO, USA)
Phosphorous Source: Orthophosphoric acid (H₃PO₄), BDH Laboratory Supplies (Poole, United Kingdom)
Crosslinking Agents: 1-Ethyl-3-(3-Dimethylaminopropyl) Carbodiimide (=EDAC), Sigma-Aldrich Inc (St. Louis, MO, USA); N-Hydroxysuccinimide (=NHS), Sigma-Aldrich Inc (St. Louis, MO, USA)

### Procedure

### Step 0: Slurry Preparation

3.8644g collagen was dispersed in 171.4mL of 0.1383M H₃PO₄ cooled in an ice bath by blending for 90 minutes at 15,000rpm using a homogeniser equipped with a 19mm diameter stator to create a highly viscous collagen dispersion. In parallel, 0.3436g chondroitin-6-sulphate (GAG) was allowed to dissolve in 14.3mL of 0.1383M H₃PO₄ at room temperature by shaking periodically to disperse dissolving GAG in order to produce a GAG solution. After 90 minutes, the 14.3mL of GAG solution was added to the mixing collagen dispersion at a rate of approximately 0.5mL/min under continuous homogenisation at 15,000rpm, and the resulting highly-viscous collagen/GAG dispersion blended for an additional 90 minutes. After 90 minutes of mixing, 1.804g Ca(OH)₂ and 0.780g Ca(NO₃)₂·4H₂O were added to the highly-viscous collagen/GAG dispersion over 30 minutes under constant blending at 15,000rpm, creating a collagen/GAG/CaP slurry, the pH of which was approximately 4.0. The collagen/GAG/CaP slurry was allowed to remain at 25°C for a period of 48 hours mixing on a stir plate, and was then placed at 4°C for a subsequent 12 hours. The chilled slurry was then degassed in a vacuum flask over 25 hours at a pressure of 25 Pa.

### Step I: Casting

15mL of the mineralised collagen/GAG/CaP slurry was cast into a polysulphone mould, 50mm long by 30mm wide by 10mm deep, using an auto-pipettor. All large bubbles were removed from the slurry using a hand pipettor.

### Step II: Inter-diffusion

As the scaffold for Example I'comprised only one layer, the inter-diffusion step was unnecessary.

### Step III: Controlled Cooling

The mould and slurry were placed in a VirTis Genesis freeze dryer (equipped with temperature-controlled, stainless steel shelves) and the shelf temperature of the freeze dryer ramped from 4°C to -20°C at a rate of approximately 2.4°C per minute.

### Step IV: Annealing

The shelf temperature of the freeze dryer was maintained at -20°C for 10 hour.

### Step V: Sublimation

While still at a shelf temperature of -20°C, a vacuum of below 25Pa (approximately 200mTorr) was applied to the chamber containing the mould and the (now frozen) slurry. The temperature of the chamber was then raised to 37°C, and sublimation allowed to continue for 36 hours. The vacuum was then removed, and the temperature returned to room temperature, leaving a single-layered scaffold of collagen/GAG/CaP, 50mm by 30mm by 10mm in size.

### Step V + I: Crosslinking

Scaffolds were hydrated in 40mL deionised water for 20 minutes. 20mL of a solution of 0.035M EDAC and 0.014M NHS was added to the container containing the scaffolds and deionised water, and the scaffolds were allowed to crosslink for 2 hours at room temperature under gentle agitation. The EDAC solution was removed, and the scaffolds were rinsed with phosphate buffer solution (PBS) and then allowed to incubate at 37°C for 2 hours in fresh PBS under mild agitation. After two hours in PBS, the scaffolds were rinsed by allowing them to incubate in deionised water for two ten-minute intervals at 37°C under mild agitation. The scaffolds were then freeze-dried to remove any residual water by controlled cooling from room temperature to -20°C at a rate of approximately 2.4°C per minute, followed by annealing at -20°C for approximately 5 hours, and then sublimation at below 25Pa at 37°C, resulting in a crosslinked collagen/GAG/CaP scaffold roughly 50mm by 30 mm by 10mm in size.

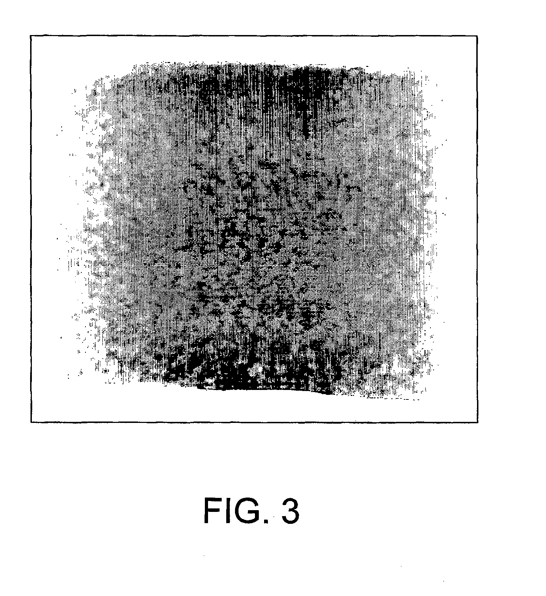

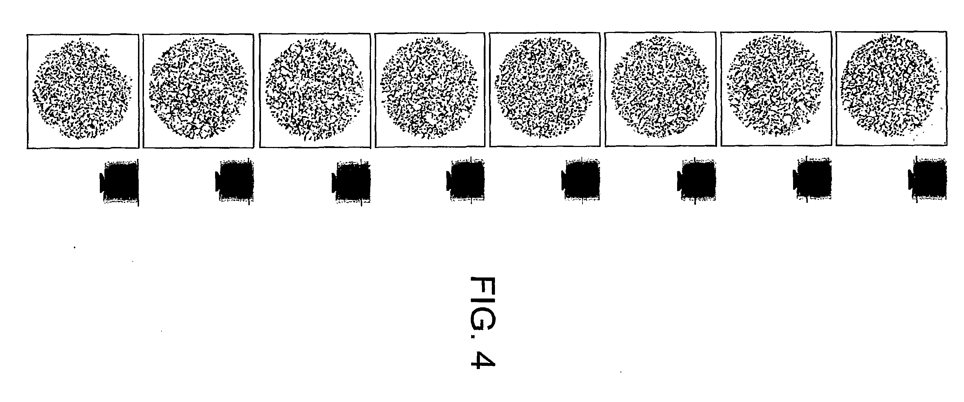

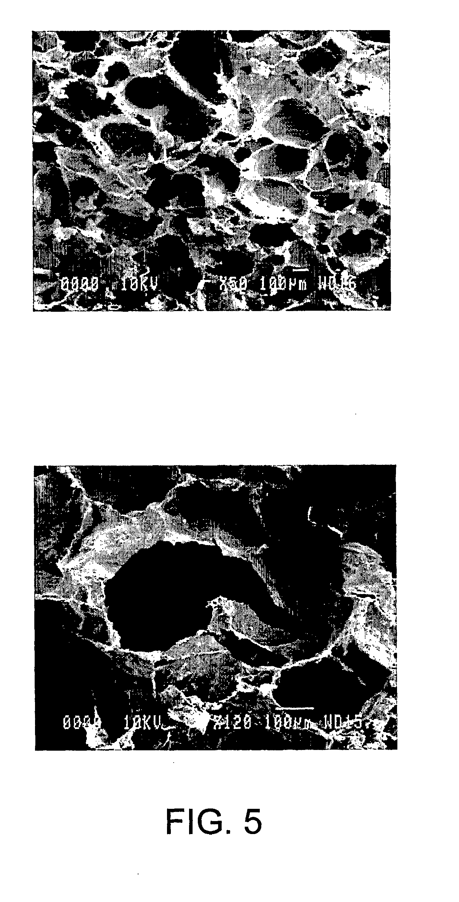

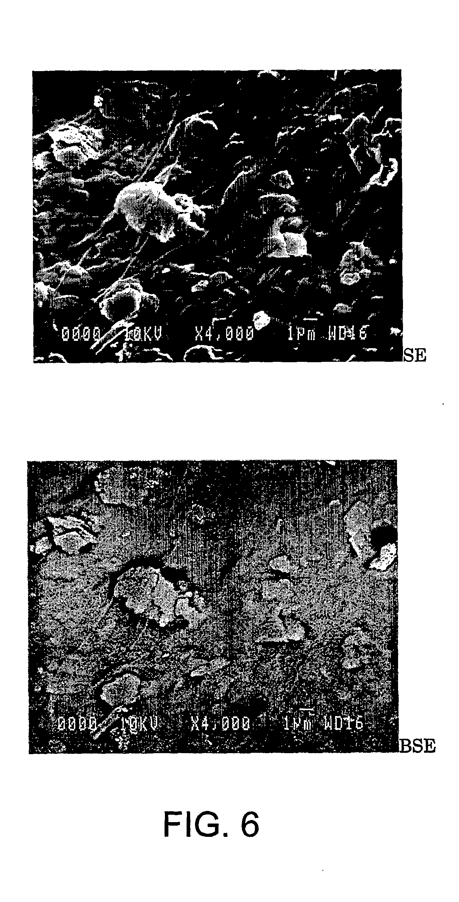

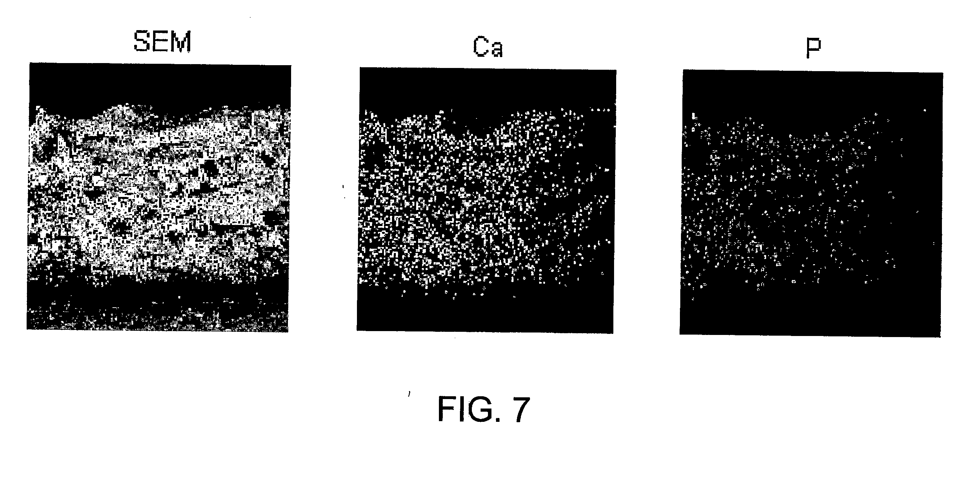

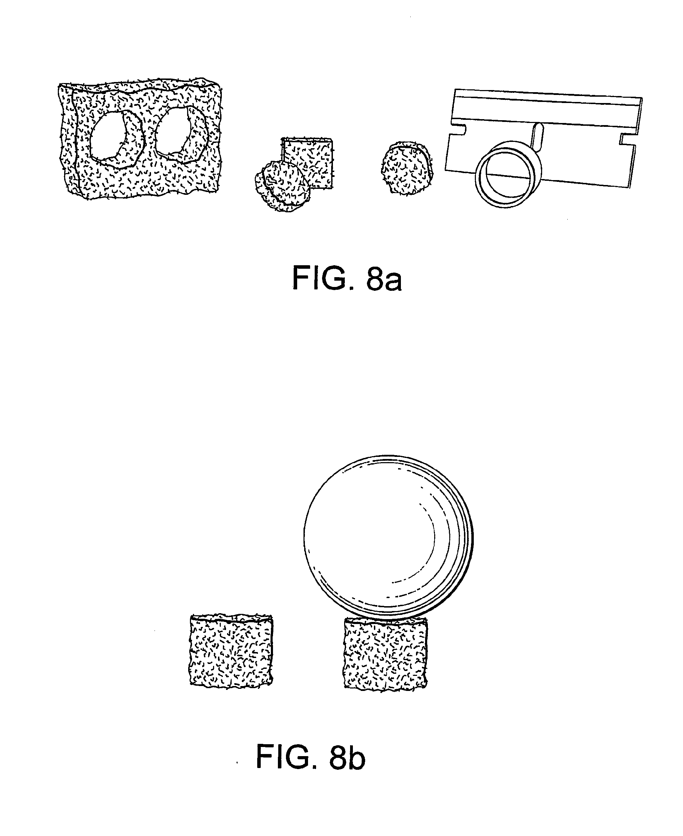

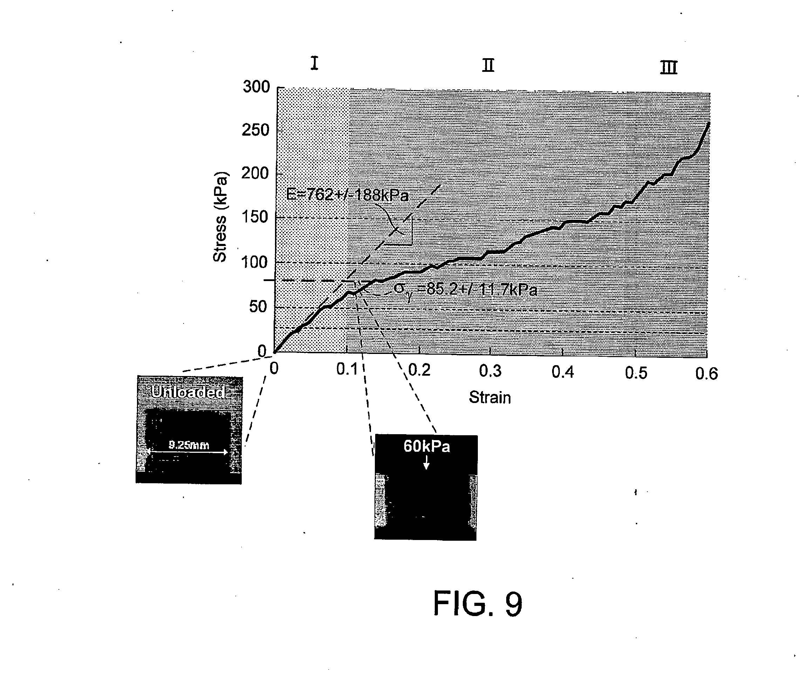

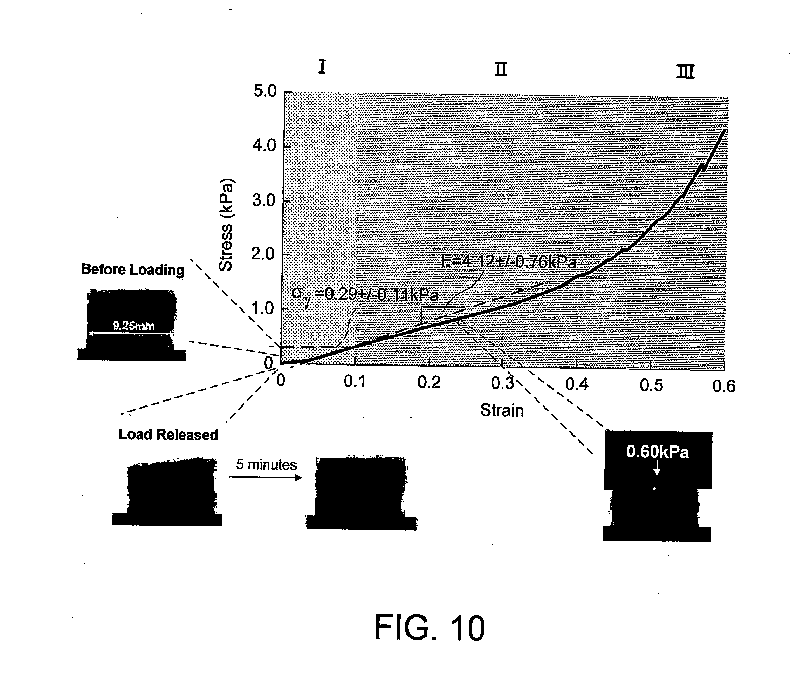

X-ray microtomographic images, scanning electron microscope images, ion distribution maps and compressive mechanical behaviour of the resulting one-layer scaffolds are shown in Figures 3 to 10. Figure 3 shows a profile of a 9.5mm x 9.5mm cylindrical section of the scaffold produced by the above procedure, as viewed through X-ray microtomography. Of note is the substantially uniform nature of both material composition and porosity throughout the scaffold. Sequential cross-sections of the same scaffold are shown in Figure 4, again illustrating the uniform nature of the scaffold pore structure; also evident in Figure 4 is the high degree of pore interconnectivity, the equiaxed pore morphology and the large (mean diameter of 500 microns) macropore size. In Figure 5, SEM micrographs again show the macropore morphology while also showing the presence of limited microporosity, visible within the walls of certain macropores. High (4000x) magnification secondary (i.e. topography-sensitive) and backscattered (i.e. composition-sensitive) electron images of a region of the scaffold wall (Figure 6) demonstrate the compositional homogeneity of the scaffold walls, despite the presence of limited topological variations in the form of protruding nodules approximately 1-2 microns in size. The calcium and phosphorous maps shown in Figure 7 corroborate the conclusion of substantially compositional homogeneity throughout the scaffold, with both elements distributed evenly throughout the scaffold. Figure 8 shows single-layered scaffolds in the dry state, and illustrates their ability to be cut to any desired shape without crumbling, cracking or losing their integrity using common surgical tools such as scalpels, razor blades and trephine blades (circular cutting tools used during corneal transplantation); Figure 8 also illustrates the weight bearing capacity of dry single-layered scaffolds under the weight of a solid-steel ball-bearing. In Figure 9, the behaviour of single-layered scaffolds in the dry state is shown. This behaviour exhibits the three-stages of deformation typical of porous solids, with an elastic modulus of 762+/-188kPa and a compressive yield stress of 85.2+/-11.7kPa. It is significant to note that the yield strength of the dry scaffolds allows them to withstand firm thumb pressure (during insertion into a defect site, for example) without deforming permanently yet still be formed when strong thumb pressure is applied (by a surgeon modifying the shape of the implant, for example). In Figure 10, the compressive deformation of single-layered scaffolds in the hydrated state is shown. As in the dry state, hydrated mineralised collagen/GAG scaffolds exhibit three-stage mechanical behaviour under compressive loading, but with elastic modulus (4.12+/-0.76kPa) and yield stress (0.29+/-0.11kPa) roughly an order of magnitude lower than the corresponding properties of dry scaffolds. Furthermore, evidence of viscoelastic strain recovery has been observed following release of compressive stresses in the collapse plateau region.

### Example II: Two-Layer Mineralised-Unmineralised Scaffold

### Materials

Collagen (for mineralised slurry): Type I microfibrillar collagen from'bovine tendon, Integra Life Sciences Plainsboro, NJ, USA
GAG (for mineralised slurry): Chondroitin-6-sulphate from shark cartilage, sodium salt, Sigma-Aldrich Inc (St. Louis, MO, USA)

### Type II Collagen

+ GAG (for unmineralised slurry): Type II Collagen and GAG (Collagen/GAG) slurry solubilised from porcine cartilage, Gelstlich Biomaterials (Wolhusen, Switzerland).

Calcium Sources: (i) Calcium hydroxide (Ca(OH)₂) Sigma-Aldrich Inc (St. Louis, MO, USA); (ii) Calcium nitrate, Ca(NO₃)₂.4H₂O, Sigma-Aldrich Inc (St. Louis, MO, USA) Phosphorous Source: Orthophosphoric acid (H₃PO₄), BDH Laboratory Supplies (Poole, United Kingdom) Crosslinking Agents: 1-Ethyl-3-(3-Dimethylaminopropyl) Carbodiimide (=EDAC), Sigma-Aldrich Inc (St. Louis, MO, USA); N-Hydroxysuccinimide (=NHS), Sigma-Aldrich Inc (St. Louis, MO, USA)

### Step 0: Slurry Preparation

### Mineralised Slurry Preparation

3.8644g collagen was dispersed in 171.4mL of 0.1383M H₃PO₄ cooled in an ice bath by blending for 90 minutes at 15,000rpm using a homogeniser equipped with a 19mm diameter stator to create a highly viscous collagen dispersion. In parallel, 0.3436g chondroitin-6-sulphate (GAG) was allowed to dissolve in 14.3mL of 0.1383M H₃PO₄ at room temperature by shaking periodically to disperse dissolving GAG in order to produce a GAG solution. After 90 minutes, the 14.3mL of GAG solution was added to the mixing collagen dispersion at a rate of approximately 0.5mL/min, under continuous homogenisation at 15,000rpm, and the resulting highly-viscous collagen/GAG dispersion blended for an additional 90 minutes. After 90 minutes of mixing, 1.804g Ca(OH)₂ and 0.780g Ca(NO₃)₂·4H₂O were added to the highly-viscous collagen/GAG dispersion over 30 minutes under constant blending at 15,000rpm, creating a collagen/GAG/CaP slurry, the pH of which was approximately 4.0. The chilled slurry was then degassed in a vacuum flask over 25 hours at a pressure of 25 Pa, reblended using the homogenizer over 30 minutes, and then degassed again for 48 hours.

### Unmineralised Slurry Preparation

Type II collagen/GAG slurry was removed from refrigerator and allowed to return to room temperature.

### Step I: Casting

2.5mL of the unmineralised Type II collagen/GAG slurry was placed in the bottom portion of a combination polysulphone mould, the bottom portion of which measured 50mm in length by 30mm in width by 2mm in depth. The slurry was smoothed to a flat surface using a razor blade. An upper collar, also made of polysulphone, and measuring 50mm in length by 30mm in width by 6mm in depth, was attached to the bottom portion of the mould containing the smoothed, unmineralised slurry. 9mL of the mineralised collagen/GAG/CaP slurry was placed, in an evenly distributed manner, on top of the

smoothed, unmineralised layer and within the previously empty upper collar. All large bubbles were removed from the slurry using a hand pipettor.

### Step II: Inter-diffusion

The layered slurry was allowed to remain at room temperature and pressure for a total of 4 hours, before being placed in the freeze dryer.

### Step III: Controlled Cooling

The mould and layered slurry were placed in a VirTis Genesis freeze dryer (equipped with temperature-controlled, stainless steel shelves) and the shelf temperature of the freeze dryer ramped from 4°C to -40°C at a rate of approximately -2.4°C per minute.

### Step IV: Annealing

The shelf temperature of the freeze dryer was maintained at -40°C for 10 hours.

### Step V: Sublimation

While still at a shelf temperature of -40°C, a vacuum of below 25Pa (approximately 200mTorr) was applied to the chamber containing the mould and the (now frozen) layered slurry. The temperature of the chamber was then raised to 37°C, and sublimation allowed to continue for 36 hours. The vacuum was then removed, and the temperature returned to room temperature, leaving a two-layered scaffold of collagen/GAG/CaP, 50mm by 30mm by 8mm in size, comprised of an unmineralised layer 2mm thick, and a mineralised layer 6mm thick.

### Step V + I: Crosslinking

Scaffolds were hydrated in 32mL deionised water for 20 minutes. 18mL of a solution of 0.035M EDAC and 0.014M NHS was added to the container containing the scaffolds and deionised water, and the_ scaffolds were allowed to crosslink for 2 hours at room temperature under gentle agitation. The EDAC solution was removed and the scaffolds were then rinsed with phosphate buffer solution (PBS) and then allowed to incubate at 37°C for 2 hours in fresh PBS under mild agitation. After two hours in PBS, the scaffolds were rinsed by allowing them to incubate in deionised water for two 10-minute intervals at 37°C under mild agitation. The scaffolds were then freeze-dried to remove any residual water by controlled cooling from room temperature to -20°C at a rate of approximately -2.4°C per minute, followed by annealing at -20°C for 5 hours, and finally by sublimation at below 25Pa at 37°C for 24 hours, resulting in a crosslinked, layered collagen/GAG/CaP scaffold roughly 50mm by 30 mm by 8mm in size, comprised of an unmineralised layer 2mm thick, and a mineralised layer 6mm thick.

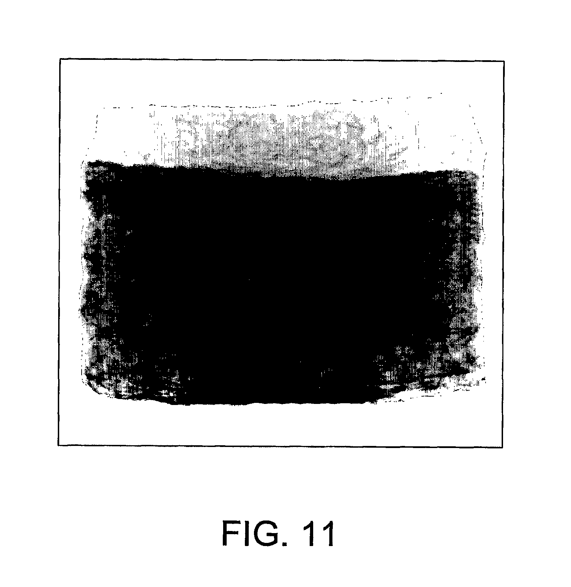

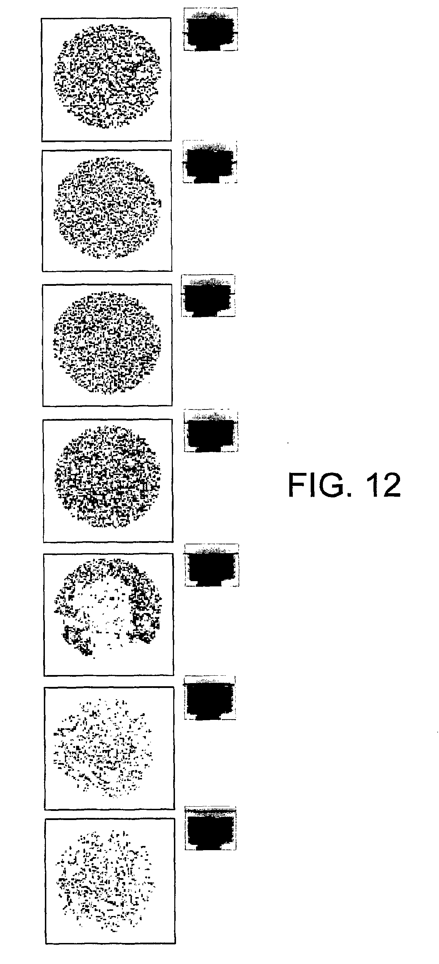

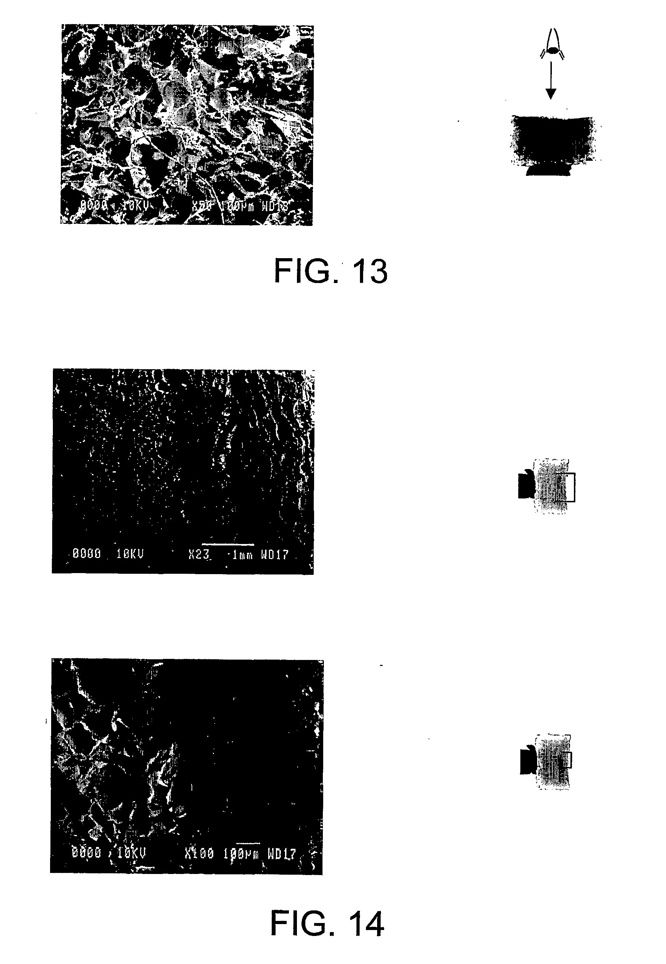

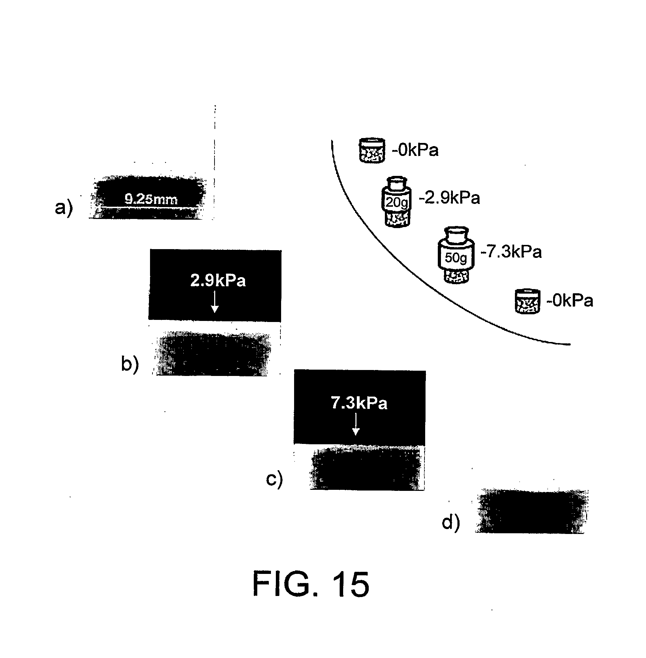

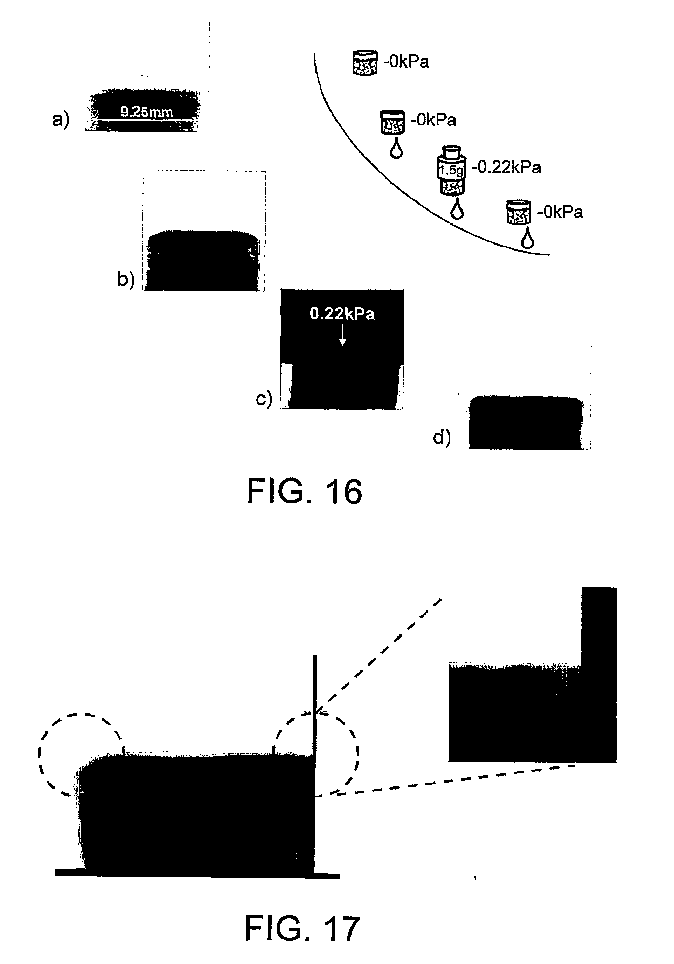

X-ray microtomographic images, scanning electron microscope images, and ion distribution maps of the resulting two-layer scaffolds are shown in Figures 11 to 17. An x-ray microtomographic image of a 9.5mm x 9.5mm cylindrical section of the two-layer scaffold produced by the procedure described above is shown in Figure 11. The opaque lower region shows the mineralised layer, while the more translucent upper region represents the unmineralised layer. It can be seen that both layers are largely uniform, both in terms of porosity and composition. The serial cross sections shown in Figure 12 show the mean macropore size in the mineralised layer to be approximately 400 microns, while that in the unmineralised layer is on the order of 700 microns; the pores in both mineralised and unmineralised layers exhibit an equiaxed morphology. The SEM image in Figure 13 shows a top view of the unmineralised layer, illustrating that little evidence of microporosity is present, while the images of the interface region shown in Figure 14 demonstrate the lack of any large voids or other discontinuities separating the mineralised and unmineralised layers. In Figure 15 the behaviour of two-layered scaffolds under compressive loading is shown. Upon application of compressive load, the compliant unmineralised layer begins to compress, resulting in near-complete compaction of the cartilaginous compartment at stresses insufficient to induce any significant deformation in mineralised scaffolds. After the load is released, the unmineralised collagen/GAG layer returns to its original shape almost instantaneously (Figure 15d). Figure 16 illustrates the mechanical behaviour of two-layered scaffolds in the hydrated state. Once hydrated, the unmineralised collagen/GAG layer can be compressed under low-magnitude loads (Figure 16a-c). Unlike in the dry state, the hydrated unmineralised compartment does not fully regain its original thickness after the first application of compressive load (Figure 16d), but instead drapes over the cross section of the mineralised compartment. After this initial compression, however, the unmineralised layer returns to its compressed thickness (Figure 16d) after each subsequent application of compressive load. In Figure 17, the ability of the unmineralised layer of a two-layer scaffold to adhere to the walls of a surgical defect encompassing the bone and cartilage interface in articular joints is illustrated by analogy. The glass slide in Figure 17 is analogous to the wall of an osteochondral defect, and the ability of the unmineralised layer to adhere to this surface illustrates the capacity of these scaffolds to fill such defects to their periphery without the persistence of gaps between the unmineralised layer of the scaffold and the adjacent articular cartilage.

### Example III: Three Layer Mineralised-Unmineralised Mineralised Scaffold

### Materials

Collagen (for mineralised slurry): Type I microfibrillar collagen from bovine tendon, Integra Life Sciences (Plainsboro, NJ, USA)
GAG (for mineralised slurry): Chondroitin-6-sulphate from shark cartilage, sodium salt, Sigma-Aldrich Inc (St. Louis, MO, USA)

Calcium Sources: (i) Calcium hydroxide (Ca(OH)₂), Sigma-Aldrich Inc (St. Louis, MO, USA); (ii) Calcium nitrate (Ca(NO₃)₂.4H₂O), Sigma-Aldrich Inc (St. Louis, MO, USA) Phosphorous Source: Orthophosphoric acid (H₃PO₄), BDH Laboratory Supplies (Poole, United Kingdom)

Collagen (for unmineralised collagen-GAG slurry): 85% Type I, 15% Type III Pepsin solubilised from porcine dermis, Japan Meat Packers (Osaka, Japan)
GAG (for unmineralised slurry): Chondroitin-6-sulphate from shark cartilage, sodium salt, Sigma-Aldrich Inc (St. Louis, MO, USA)

Diluents for unmineralised Collagen and GAG: Glacial acetic acid (CH₃COOH), Fischer Scientific (Loughborough, UK) Crosslinking Agents: Nordihydroguariaretic acid (NDGA), Sigma-Aldrich Inc (St. Louis, MO, USA);
Sodium dihydrogen phosphate (NaH₂PO₄), BDH Laboratory Supplies (Poole, United Kingdom)
Sodium chloride (NaCl), Sigma-Aldrich Inc (St. Louis, MO, USA)

### Step 0: Slurry Preparation

### Mineralised Slurry Preparation

3.8644g collagen was dispersed in 171.4mL of 0.1383M H₃PO₄ cooled in an ice bath by blending for 90 minutes at 15,000rpm, using a homogeniser equipped with a 19mm diameter stator to create a highly viscous collagen dispersion. In parallel, 0.3436g chondroitin-6-sulphate (GAG) allowed to dissolve in 14.3mL of 0.1383M H₃PO₄ at room temperature by shaking periodically to disperse dissolving GAG in order to produce a GAG solution. After 90 minutes, the 14.3mL of GAG solution was added to the mixing collagen dispersion at a rate of approximately 0.5mL/min, under continuous homogenisation at 15,000rpm, and the resulting highly-viscous collagen/GAG dispersion blended for an additional 90 minutes. After 90 minutes of mixing, 1.804g Ca(OH)₂ and 0.780g Ca(NO₃)₂·4H₂O were added to the highly-viscous collagen/GAG dispersion over 30 minutes under constant blending at 15,000rpm, creating a collagen/GAG/CaP slurry, the pH of which was approximately 4.0. The chilled slurry was then degassed in a vacuum flask over 25 hours at a pressure of 25Pa, reblended using the homogenizer over 30 minutes, then degassed again for 48 hours.

### Unmineralised Slurry Preparation

1.9322g of the Type I/III collagen was dispersed in 171.4mL of 0.05M acetic acid cooled in an ice bath by blending for 90 minutes at 15,000rpm, using a homogeniser equipped with a 19mm diameter stator in order to create a highly viscous collagen dispersion. In parallel, 0.1718g chondroitin-6-sulphate (GAG) was allowed to dissolve in 28.6mL of 0.05M acetic acid at room temperature, by shaking periodically to disperse dissolving GAG in order to produce a GAG solution. After 90 minutes, the 14.3mL of GAG solution was added to the mixing collagen dispersion at a rate of approximately 0.5mL/min, under continuous homogenisation at 15,000rpm, and the resulting highly-viscous collagen/GAG dispersion blended for an additional 90 minutes.

### Step I: Casting

3.5mL of the mineralised collagen/GAG/CaP slurry was placed in the bottom portion of a combination polysulphone mould, the bottom portion of which measured 50mm in length by 30mm in width by 3mm in depth. The slurry was smoothed to a flat surface using a razor blade. A middle collar, also made of polysulphone, and measuring 50mm in length by 30mm in width by 5mm in depth, was attached to the bottom portion of the mould containing the smoothed, mineralised slurry. 7.5mL of the unmineralised collagen/GAG slurry was placed, in an evenly distributed manner, on top of the smoothed, unmineralised layer and within the previously empty middle collar. An upper collar, also made of polysulphone and measuring 50mm in length by 30mm in width by 3mm in depth, was attached to the middle portion of the mould above the smoothed, unmineralised slurry. 3.5mL of the mineralised collagen/GAG/CaP slurry was placed, in an evenly distributed manner, on top of the smoothed, unmineralised layer and within the previously empty upper collar. All large bubbles were removed from the slurry using a hand pipettor

### Step II: Inter-diffusion

The three-layer slurry was allowed to remain at room temperature and pressure for 20 minutes before being placed in the freeze dryer.

### Step III: Controlled Cooling

The mould and three-layer slurry were placed in a VirTis Advantage freeze dryer (equipped with temperature-controlled, stainless steel shelves) and the shelf temperature of the freeze dryer ramped from 4°C to -40°C at a rate of approximately -2.4°C per minute.

### Step IV: Annealing

The shelf temperature of the freeze dryer was maintained at -40°C for 10 hours.

### Step V: Sublimation

While still at a shelf temperature of -40°C, a vacuum of below 25Pa (approximately 200mTorr) was applied to the chamber containing the mould and the (now frozen) three-layer slurry. The temperature of the chamber was then raised to 37°C, and sublimation allowed to continue for 36 hours. The vacuum was then removed, and the temperature returned to room temperature, leaving a three-layered scaffold 50mm by 30mm by 11mm in size, comprised of an unmineralised middle layer 5mm thick, surrounded by two mineralised layers 3mm thick.

### Step VI: Crosslinking

The three-layer scaffold was hydrated in 0.1M NaH2PO4 and 0.15M NaCl in phosphate buffered saline (PBS; pH 7.0) for 30 minutes. NDGA was suspended in 1N NaOH and added to PBS to produce a 3mg/mL solution of NGDA in PBS; scaffolds were then hydrated in this solution under agitation for 24 hours. The three-layer scaffold was removed from the NGDA-PBS solutions and rinsed with deionised water. The scaffolds were then freeze-dried to remove any residual water by controlled cooling from room temperature to -20°C at a rate of approximately 2.4°C per minute, followed by annealing at -20°C for 5 hours, and finally sublimation at below 25Pa at 37°C for 24 hours, resulting in a dry, crosslinked scaffold. A subsequent treatment was then performed at a concentration of 0.1mg/mL NDGA. The scaffolds were then washed in 70% ethanol for 6 hours and subsequently washed for 24 hours in PBS at room temperature. The scaffolds were then freeze dried for a second time to remove any residual water by controlled cooling from room temperature to -20°C at a rate of approximately 2.4°C per minute, followed by annealing at -20°C for 5 hours, and finally sublimation at below 25Pa at 37°C for 24 hours.

The parameters in the Tables below are applicable singularly or in combination to any aspect of the present invention unless otherwise stated.

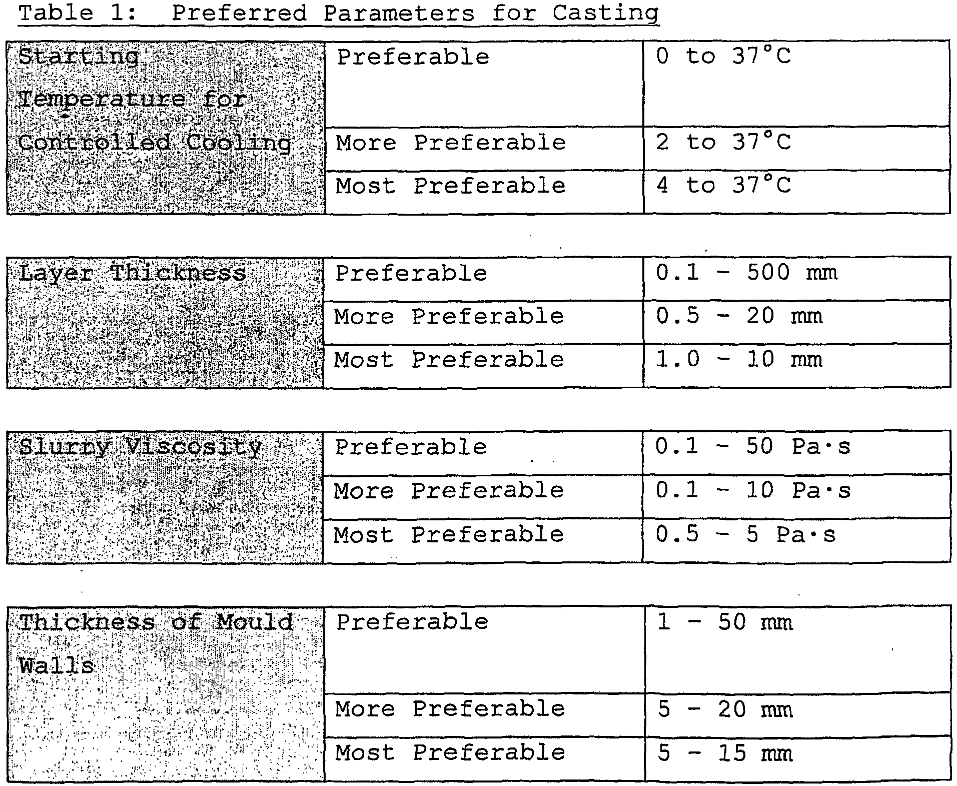

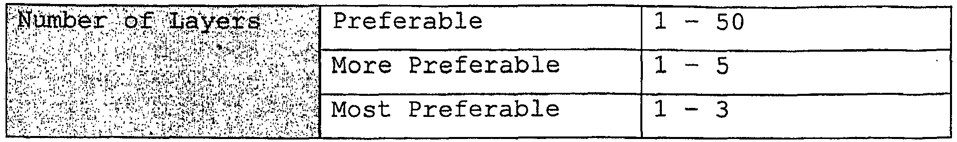

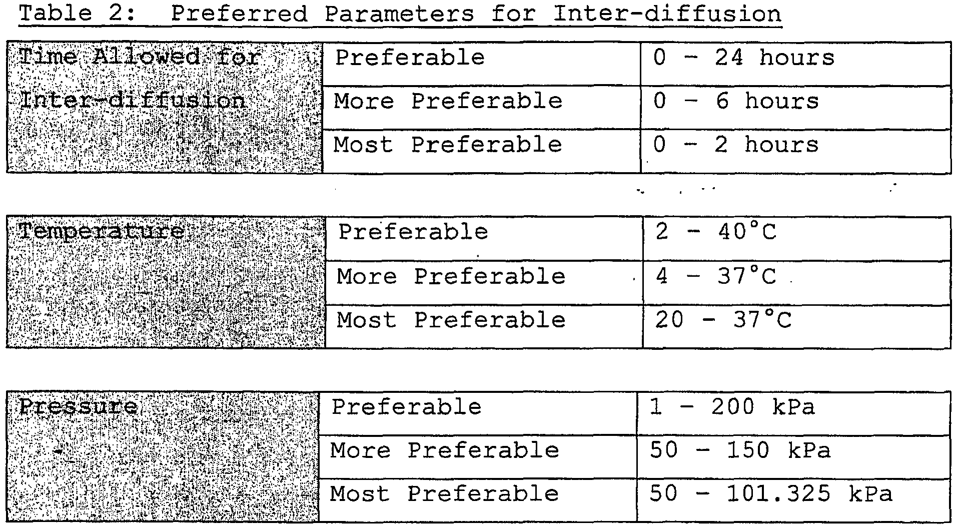

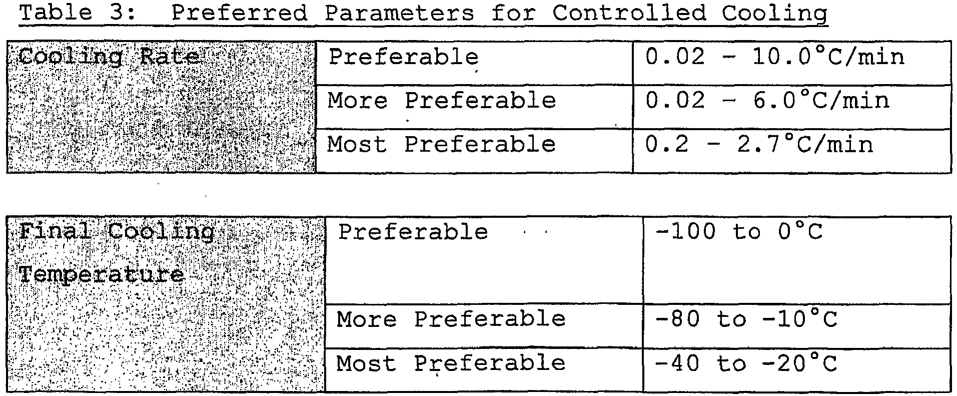

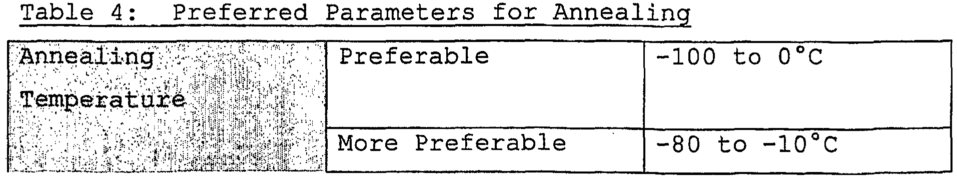

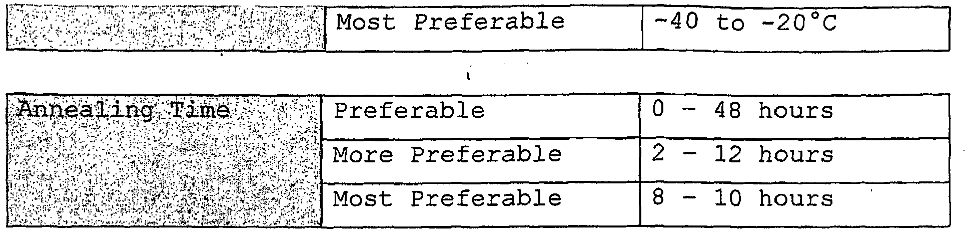

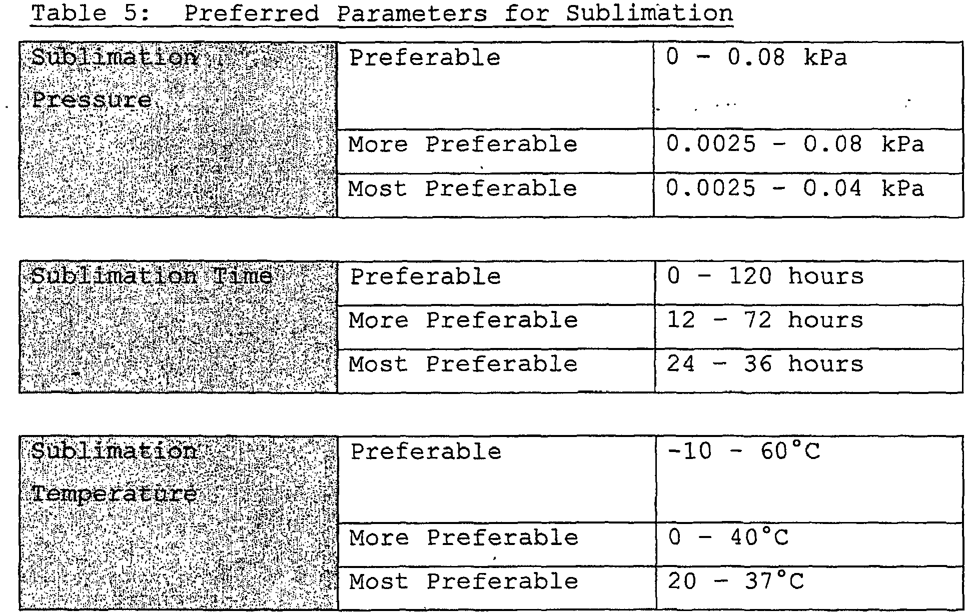

### References

Gao J, Dennis JE, Solchaga LA, Awadallah AS, Goldberg VM, Caplan AI. 2001. Tissue-Engineered Fabrication of an Osteochondral Composite Graft Using Rat Bone Marrow-Derived Mesenchymal Stem Cells. Tissue Engineering 7:363-371.
Gao J, Dennis JE, Solchaga LA, Goldberg VM, Caplan AI. 2002. Repair of Osteochondral Defect with Tissue-Engineered Two-Phase Composite Material of Injectable Calcium Phosphate and Hyaluronan Sponge. Tissue Engineering 8:827-837.
Hung CT, Lima EG, Mauck RL, Taki E, LeRoux MA, Lu HH, Stark RG, Guo XE, Ateshian GA. 2003. Anatomically Shaped Osteochondral Constructs for Articular Cartilage Repair. Journal of Biomechanics 36:1853-1864.
Hunziker EB, Driesang IMK. 2003. Functional Barrier Principle for Growth-Factor-Based Articular Cartilage Repair. Osteoarthritis and Cartilage 11:320-327.
Niederauer GG, Slivka MA, Leatherbury NC, Korvick DL, H.H. HJ, Ehler WC, Dunn CJ, Kieswetter K. 2000. Evaluation of Multiphase Implants for Repair of Focal Osteochondral Defects in Goats. Biomaterials 21:2561-2574.
O'Brien FJ, Harley BA, Yannas IV, Gibson L. 2004. Influence of Freezing Rate on Pore Structure in Freeze-Dried Collagen-GAG Scaffolds. Biomaterials 25:1077-1086.
O'Brien FJ, Harley BA, Yannas IV, Gibson LJ. 2005. The Effect of Pore Size and Structure on Cell Adhesion in Collagen-GAG Scaffolds. Biomaterials 26:433-441.
HM Loree, IV Yannas, B Mikic, AS Chang, SM Perutz, TV Norregaard, and C Kararup, 'A freeze-drying process for fabrication of polymeric bridges for peripheral nerve regeneration' Proc. 15th Annual Northeast Bioeng. Conf. P.53-54, 1989.
Schaefer D, Martin I, Jundt G, Seidel J, Heberer M, Grodzinsky A, Bergin I, Vunjak-Novakovic G, Freed LE. 2002. Tissue-Engineered Composites for the Repair of Large Osteochondral Defects. Arthritis and Rheumatism 46:2524-2534.
Schaefer D, Martin I, Shastri P, Padera RF, Langer R, Freed LE, Vunjak-Novakovic G. 2000. In Vitro Generation of Osteochondral Composites. Biomaterials 21:2599-2606.
Sherwood JK, Riley SL, Palazzolo R, Brown SC, Monkhouse DC, Coates M, Griffith LG, Landeen LK, Ratcliffe A. 2002. A Three-Dimensional Osteochondral Composite Scaffold for Articular Cartilage Repair. Biomaterials 23:4739-4751.
Yannas IV, Lee E, Orgill DP, Skrabut EM, Murphy GF. 1989. Synthesis and Characterization of a Model Extracellular Matrix that Induces Partial Regeneration of Adult Mammalian Skin. Proceedings of the National Academy of Sciences of the United States of America 86:933-937.

The present invention finds application in a number of areas and the following are provided by way of example.

### Articular Cartilage Repair Product: Two-Layer Scaffold

Two layer scaffolds hold the potential to enhance the efficacy of existing first-line surgical procedures that recruit marrow-derived stem cells to the site of articular-cartilage injury. Delivered as, for example, a dry, 2cm x 2cm x 1cm block of dry, vacuum-packed, gamma-sterilised material resembling styrofoam, these scaffolds can be cut using a scalpel or other tools, are easily inserted into the defect using simple thumb- or blunt-instrument pressure, and bond directly to the site without sutures or glue.

### Patellar Ligament Donor-Site Repair Product: Three Layer Scaffolds

Three-layer scaffolds hold the potential to enhance regeneration at patellar ligament (patella tendon) donor sites during anterior cruciate ligament (ACL) reconstruction, reducing frontal knee pain and reducing the risk of patellar ligament rupture and patellar fracture.

### Tendon Repair Product: Two- Layer Scaffolds

Two-layer scaffolds with extended unmineralised components hold the potential to improve the efficacy of tendon repair during rotator-cuff procedures and to address small-tendon applications for which no effective solution currently exists.

The present invention has been further studied on the basis of large-animal trials and a summary is presented below.

### Trial 1: Ovine Bone Defect Model

The present invention enables the production of layered tissue regeneration scaffolds whose structure and composition mimic bone on one side, unmineralised tissue (e.g. cartilage, ligament, tendon) on the other side, and a smooth, stable interface in between. The present invention furthermore offers the capacity to systematically alter the chemical composition of the mineral phase of the bony compartment of such implants.
Animal: skeletally mature Texcel Continental sheep (female).
Defect: 9mm diameter by 9mm deep cancellous bone defect on lateral femoral condyle.
Implantation Period: 6 weeks.
Experimental Groups: Six implants of each experimental group implanted contralaterally with the same implant type in each side of the same animal.

### Control Groups:

Positive Control: four sites were filled with cancellous autograft harvested from the tibial tuberosity.
Negative Control: four sites were filled with control implants comprising implants containing no mineral phase at all (i.e. containing the organic constituents of the bony side of ChondroMimetic only).
Study Objective: to identify differences in the performance of four experimental implant groups differentiated by chemical composition and to identify the most desirable of these as the final composition for the bone compartment of ChondroMimetic.
Significant Findings: none of the three experimental groups invoked adverse immune responses of any kind; all three experimental groups plus the unmineralised negative control group supported bony in-growth via a cell-mediated direct substitution mechanism; no statistically significant differences between there three implant groups were observed; and bone formation observed in all three experimental groups was higher than that in the negative control group to a statistically significant level.
Implications for Implant Design: The direct substitution mechanism implied by this study suggests that the bone formation mechanism more closely resembles the templated bone formation that occurs at the growth plate in foetal and neonatal animals (including humans) than the typical apposition mechanism observed in traditional bone-graft substitutes. The presence of this substitution mechanism in the unmineralised control suggests that it is the organic constituent of the implants that imparts this character.

Pore size for the implants should be altered to account for this substitution mechanism by reducing the mean pore size of the bony compartment of the implants.

Lack of statistically significant differences in the bone formation behaviour of the three experimental groups suggests that processing parameters may be used to identify the most appropriate mineral composition of the implants.

### Trial 2: Caprine Osteochondral Defect Model

The objective of this study was to evaluate the performance of ChondroMimetic as a means of improving the results of a marrow stimulation technique (subchondral drilling).
Animal: skeletally mature Spanish goats (female).
Defect: 4mm diameter by 6mm deep osteochondral defects (1 in trochlear groove; 1 on the lateral condyle).
Implantation Period: 16 weeks.
Experimental Groups: Six implants of the ChondroMimetic working prototype.
Control Group: Six defects simulating traditional subchondral drilling (i.e. containing no implants).
Study Objective: to evaluate the performance of ChondroMimetic as an aid to marrow stimulation
Findings: feedback from surgeons about the handling characteristics of ChondroMimetic was, without exception, overwhelmingly positive.

## Claims

1. A process for the preparation of a composite biomaterial suitable for repairing interfaces between tissues comprising collagen and a calcium phosphate material, or collagen, a calcium phosphate material and a glycosaminoglycan, the process comprising:
(a) providing a first slurry composition comprising a liquid carrier, collagen, and a calcium phosphate material; and providing a second slurry composition comprising a liquid carrier and collagen; or
providing a first slurry composition comprising a liquid carrier, collagen, and a calcium phosphate material, and providing a second slurry composition comprising a liquid carrier, collagen, and a calcium phosphate material; or
providing a first slurry composition comprising a liquid carrier, collagen, and a calcium phosphate material; and providing a second slurry composition comprising a liquid carrier, collagen, a calcium phosphate material, and a glycosaminoglycan; or
providing a first slurry composition comprising a liquid carrier, collagen, a calcium phosphate material, and a glycosaminoglycan; and providing a second slurry composition comprising a liquid carrier and collagen; or
providing a first slurry composition comprising a liquid carrier, collagen, a calcium phosphate material, and a glycosaminoglycan; and providing a second slurry composition comprising a liquid carrier, collagen, and a calcium phosphate material; or
providing a first slurry composition comprising a liquid carrier, collagen, a calcium phosphate material, and a glycosanlinoglycan; and providing a second slurry composition comprising a liquid carrier, collagen, a calcium phosphate material, and a glycosaminoglycan;
(b) providing a mould for the slurry;
(c) depositing the first and second slurries in the mould;
(d) cooling the slurry deposited in the mould to a temperature at which the liquid carrier transforms into a plurality of solid crystals or particles;
(e) removing at least some of the plurality of solid crystals or particles by sublimation and/or evaporation to leave a porous composite material comprising collagen, a calcium phosphate material, and optionally a glycosaminoglycan; and
(f) removing the material from the mould.

2. A process as claimed in claim 1, wherein the first slurry comprises a co-precipitate of collagen and the calcium phosphate material or a triple co-precipitate of collagen, a glycosaminoglycan and a calcium phosphate material; and the second slurry comprises a coprecipitate of collagen and a glycosaminoglycan, a co-precipitate of collagen and the calcium phosphate material or a triple co-precipitate of collagen, a glycosaminoglycan and a calcium phosphate material.

3. A process as claimed in claim 2, wherein the first slurry and/or second slurry comprises a triple co-precipitate of collagen, the calcium phosphate material and a glycosaminoglycan.

4. A process as claimed in any one of claims 1 to 3, wherein the calcium phosphate material comprises brushite.

5. A process as claimed in any one of the preceding claims further comprising:
providing a further slurry composition comprising a liquid carrier and collagen; or
providing a further slurry composition comprising a liquid carrier, collagen, and a calcium phosphate material; or
providing a further slurry composition comprising a liquid carrier, collagen, a calcium phosphate material, and a glycosaminoglycan; and
prior to said cooling step, depositing said further slurry composition in the mould either before or after said first slurry composition has been deposited.

6. A process as claimed in any one of the preceding claims, wherein steps (d) and (e) are effected by a freeze-drying technique.

7. A process as claimed in any one of the preceding claims, wherein the liquid carrier comprises water.

8. A process as claimed in any one of the preceding claims, wherein the temperature of the slurry deposited in the mould prior to the cooling step is in the range of from 2 to 40°C, or from 4 to 37°C, or from 20 to 37°C.

9. A process as claimed in any one of the preceding claims, wherein the step of cooling the slurry deposited in the mould is carried out to a temperature in the range of from -100 to 0°C, or from -80 to -10°C, or from -40 to -20°C.

10. A process as claimed in any one of the preceding claims, wherein the step of removing at least some of the solid crystals or particles by sublimation is carried out at a pressure of from 0 - 0.08 kPa, or from 0.0025 - 0.08 kPa, or from 0.0025 - 0.04 kPa.

11. A process as claimed in any one of the preceding claims, wherein the step of removing at least some of the solid crystals or particles by sublimation is carried out for up to 96 hours, or from 12 - 72 hours, or from 24 - 36 hours.

12. A process as claimed in any one of the preceding claims, wherein the step of removing at least some of the solid crystals or particles by sublimation is carried out at a temperature of from -10 to 60°C, or from 0 to 40°C, or from 20 to 37°C, or from 25 to 37°C.

13. A process as claimed in any one of the preceding claims, further comprising the step of cross-linking the collagen and the one or more glycosaminoglycans in the porous composite biomaterial.

14. A process as claimed in any one of the preceding claims, further comprising the step of converting at least some of the calcium phosphate material in the porous composite biomaterial to a different calcium phosphate phase.

15. A synthetic composite biomaterial suitable for repairing interfaces between tissues comprising:
a first layer formed of a porous material comprising collagen and a calcium phosphate material with the proviso that the calcium phosphate material is not hydroxylapatite; and a second layer joined to the first layer and formed of a material comprising collagen; or
a first layer formed of a porous material comprising collagen and a calcium phosphate material; and a second layer joined to the first layer and formed of a material comprising a coprecipitate of collagen and a glycosaminoglycan; or
a first layer formed of a porous material comprising collagen and a calcium phosphate material; and a second layer joined to the first layer and formed of a material comprising a co-precipitate of collagen and a calcium phosphate material; or
a first layer formed of a porous material comprising collagen and a calcium phosphate material; and a second layer joined to the first layer and formed of a material comprising a triple co-precipitate of collagen, a glycosaminoglycan and a calcium phosphate material; or
a first layer formed of a porous material comprising collagen, a calcium phosphate material and a glycosaminoglycan; and a second layer joined to the first layer and formed of a material comprising collagen; or
a first layer formed of a porous material comprising collagen, a calcium phosphate material and a glycosaminoglycan; and a second layer joined to the first layer and formed of a material comprising a co-precipitate of collagen and a glycosaminoglycan; or
a first layer formed of a porous material comprising collagen, a calcium phosphate material and a glycosaminoglycan; and a second layer joined to the first layer and formed of a material comprising a co-precipitate of collagen and a calcium phosphate material; or
a first layer formed of a porous material comprising collagen, a calcium phosphate material and a glycosaminoglycan; and a second layer joined to the first layer and formed of a material comprising a triple co-precipitate of collagen, a glycosaminoglycan and a calcium phosphate material.

16. A synthetic composite biomaterial as claimed in claim 15, wherein at least part of the biomaterial is formed from a porous co-precipitate comprising collagen and a calcium phosphate material, wherein the macropore size range (pore diameter) is from 1 - 1200 microns, or from 10 -1000 microns, or from 100 - 800 microns, or from 200 - 600 microns.

17. A synthetic composite biomaterial as claimed in claim 15 or claim 16, comprising a first layer formed of a porous material comprising a triple co-precipitate of collagen, a calcium phosphate material and a glycosaminoglycan; and a second layer joined to the first layer and formed of a material comprising a co-precipitate of collagen and a glycosaminoglycan.

18. A synthetic composite biomaterial as claimed in any one of claims 15 to 17 comprising one or more further layers joined to the first and/or second layers, each of said further layers being formed of a material comprising collagen, or a co-precipitate of collagen and a glycosaminoglycan, or a co-precipitate of collagen and a calcium phosphate material, or a triple co-precipitate of collagen, a glycosaminoglycan, and at least one calcium phosphate material.

19. A synthetic composite biomaterial as claimed in any one of claims 15 to 18, wherein the first, second and said one or more further layers are integrally formed by liquid phase co-synthesis.

20. A synthetic composite biomaterial as claimed in any one of claims 15 to 19, wherein adjacent layers are joined to one another through an inter-diffusion layer.

21. A synthetic composite biomaterial as claimed in any one of claims 15 to 17, wherein the first and second layers are joined to one another through an inter layer.

22. A synthetic composite biomaterial as claimed in any one of claims 15 to 21 wherein the calcium phosphate material is selected from one or more of brushite, octacalcium phosphate and/or apatite.

23. A synthetic composite biomaterial as claimed in any one of claims 15 to 22, wherein the biomaterial comprises collagen and a glycosaminoglycan, and wherein the collagen and the glycosaminoglycan are crosslinked.

24. A synthetic composite biomaterial as claimed in any one of claims 15 to 23, wherein the collagen is present in the material in an amount of from 1 to 99 wt%, from 5 to 90 wt%, or from 15 to 60 wt%.

25. A synthetic composite biomaterial as claimed in any one of claims 15 to 24, wherein the glycosaminoglycan is present in the material in an amount of from 0.01 to 20 wt%, or from 1 to 5.5 wt%.

26. A synthetic bone material, bone implant, bone graft, bone substitute, bone scaffold, filler, coating or cement comprising a synthetic composite biomaterial as declined in any one of claims 15 to 25.

27. A layered bone scaffold for use in tissue engineering comprising a synthetic composite biomaterial as defined in one of claims 15 to 25.

## Patentansprüche

1. Verfahren zur Herstellung eines Verbund-Biomaterials, geeignet zur Herstellung von Grenzflächen zwischen Geweben, Kollagen und ein Calciumphosphatmaterial oder Collagen, ein Calciumphosphatmaterial und ein Glycosaminoglycan beinhaltend, wobei das Verfahren umfasst:
(a) Bereitstellen einer ersten Aufschlämmungszusammensetzung, die einen flüssigen Träger, Collagen und ein Calciumphosphatmaterial beinhaltet; und Bereitstellen einer zweiten Aufschlämmungszusammensetzung, die einen flüssigen Träger und Collagen beinhaltet; oder
Bereitstellen einer ersten Aufschlämmungszusammensetzung, die einen flüssigen Träger, Collagen und ein Calciumphosphatmaterial beinhaltet, und Bereitstellen einer zweiten Aufschlämmungszusammensetzung, die einen flüssigen Träger, Collagen und ein Calciumphosphatmaterial beinhaltet; oder
Bereitstellen einer ersten Aufschlämmungszusammensetzung, die einen flüssigen Träger, Collagen und ein Calciumphosphatmaterial beinhaltet; und Bereitstellen einer zweiten Aufschlämmungszusammensetzung, die einen flüssigen Träger, Collagen, ein Calciumphosphatmaterial und ein Glycosaminoglycan beinhaltet; oder
Bereitstellen einer ersten Aufschlämmungszusammensetzung, die einen flüssigen Träger, Collagen, ein Calciumphosphatmaterial und ein Glycosaminoglycan beinhaltet; und Bereitstellen einer zweiten Aufschlämmungszusammensetzung, die einen flüssigen Träger und Collagen beinhaltet; oder
Bereitstellen einer ersten Aufschlämmungszusammensetzung, die einen flüssigen Träger, Collagen, ein Calciumphosphatmaterial und ein Glycosaminoglycan beinhaltet; und Bereitstellen einer zweiten Aufschlämmungszusammensetzung, die einen flüssigen Träger, Collagen und ein Calciumphosphatmaterial beinhaltet; oder
Bereitstellen einer ersten Aufschlämmungszusammensetzung, die einen flüssigen Träger, Collagen, ein Calciumphosphatmaterial und ein Glycosaminoglycan beinhaltet; und Bereitstellen einer zweiten Aufschlämmungszusammensetzung, die einen flüssigen Träger, Collagen, ein Calciumphosphatmaterial und ein Glycosaminoglycan beinhaltet;
(b) Bereitstellen einer Form für die Aufschlämmung;
(c) Einbringen der ersten und zweiten Aufschlämmungen in die Form;
(d) Kühlen der Aufschlämmung, die in die Form eingebracht worden ist, auf eine Temperatur, bei der sich der flüssige Träger in eine Mehrzahl von festen Kristallen oder Teilchen umbildet;
(e) Entfernen von zumindest einigen von der Mehrzahl von festen Kristallen oder Teilchen durch Sublimation und/oder Verdampfung, um ein poröses Verbundmaterial zurückzubehalten, das Collagen, ein Calciumphosphatmaterial und optional ein Glycosaminoglycan beinhaltet; und
(f) Entnehmen des Materials aus der Form.

2. Verfahren nach Anspruch 1, wobei die erste Aufschlämmung ein Copräzipitat aus Collagen und dem Calciumphosphatmaterial oder ein dreifaches Copräzipitat aus Collagen, einem Glycosaminoglycan und einem Calciumphosphatmaterial beinhaltet; und die zweite Aufschlämmung ein Copräzipitat aus Collagen und einem Glycosaminoglycan, ein Copräzipitat aus Collagen und dem Calciumphosphatmaterial oder ein dreifaches Copräzipitat aus Collagen, einem Glycosaminoglycan und einem Calciumphosphatmaterial beinhaltet.

3. Verfahren nach Anspruch 2, wobei die erste Aufschlämmung und/oder die zweite Aufschlämmung ein dreifaches Copräzipitat aus Collagen, dem Calciumphosphatmaterial und einem Glycosaminoglycan beinhaltet.

4. Verfahren nach einem der Ansprüche 1 bis 3, wobei das Calciumphosphatmaterial Brushit beinhaltet.

5. Verfahren nach einem der vorangehenden Ansprüche, ferner umfassend:
Bereitstellen einer weiteren Aufschlämmungszusammensetzung, die einen flüssigen Träger und Collagen beinhaltet; oder
Bereitstellen einer weiteren Aufschlämmung, die einen flüssigen Träger, Collagen und ein Calciumphosphatmaterial beinhaltet; oder
Bereitstellen einer weiteren Aufschlämmungszusammensetzung, die einen flüssigen Träger, Collagen, ein Calciumphosphatmaterial und ein Glycosaminoglycan beinhaltet; und
vor dem Kühlungsschritt, Einbringen der weiteren Aufschlämmungszusammensetzung in die Form entweder bevor oder nachdem die erste Aufschlämmungszusammensetzung eingebracht wird bzw. worden ist.

6. Verfahren nach einem der vorangehenden Ansprüche, wobei die Schritte (d) und (e) anhand einer Gefriertrocknungstechnik durchgeführt werden.

7. Verfahren nach einem der vorangehenden Ansprüche, wobei der flüssige Träger Wasser beinhaltet.

8. Verfahren nach einem der vorangehenden Ansprüche, wobei die Temperatur der Aufschlämmung, die in die Form eingebracht worden ist, vor dem Schritt des Kühlens im Bereich von 2 bis 40 °C oder von 4 bis 37 °C oder von 20 bis 37 °C liegt.

9. Verfahren nach einem der vorangehenden Ansprüche, wobei der Schritt des Kühlens der Aufschlämmung, die in die Form eingebracht worden ist, bei einer Temperatur im Bereich von -100 bis 0 °C oder von -80 bis -10 °C oder von -40 bis -20 °C durchgeführt wird.

10. Verfahren nach einem der vorangehenden Ansprüche, wobei der Schritt des Entfernens zumindest einiger von den festen Kristallen oder Teilchen durch Sublimation bei einem Druck ausgeführt wird, der bei 0-0,08 kPa oder bei 0,0025 - 0,08 kPa oder bei 0,0025 - 0,04 kPa liegt.

11. Verfahren nach einem der vorangehenden Ansprüche, wobei der Schritt des Entfernens zumindest einiger von den festen Kristallen oder Teilchen durch Sublimation für bis zu 96 Stunden oder für 12 - 72 Stunden oder für 24 - 36 Stunden durchgeführt wird.

12. Verfahren nach einem der vorangehenden Ansprüche, wobei der Schritt des Entfernens zumindest einiger von den festen Kristallen oder Teilchen durch Sublimation bei einer Temperatur von -10 bis 60 °C oder von 0 bis 40 °C oder von 20 bis 37 °C oder von 25 bis 37 °C durchgeführt wird.

13. Verfahren nach einem der vorangehenden Ansprüche, ferner den Schritt des Vernetzens des Collagens und des mindestens einen Glycosaminoglycans in dem porösen Verbund-Biomaterial umfassend.

14. Verfahren nach einem der vorangehenden Ansprüche, ferner den Schritt des Umwandelns zumindest eines Teils des Calciumphosphatmaterials in dem porösen Verbund-Biomaterial in eine andere Calciumphosphatphase umfassend.

15. Synthetisches Verbund-Biomaterial, das geeignet ist, um Grenzflächen zwischen Geweben zu reparieren, aufweisend:
eine erste Schicht, die aus einem porösen Material gebildet ist, das Collagen und ein Calciumphosphatmaterial beinhaltet, mit der Maßgabe, dass das Calciumphosphatmaterial nicht Hydroxylapatit ist; und eine zweite Schicht, die an die erste Schicht angefügt ist und aus einem Material gebildet ist, das Collagen beinhaltet; oder
eine erste Schicht, die aus einem porösen Material gebildet ist, das Collagen und ein Calciumphosphatmaterial beinhaltet; und eine zweite Schicht, die an die erste Schicht angefügt ist und aus einem Material gebildet ist, das ein Copräzipitat aus Collagen und einem Glycosaminoglycan beinhaltet; oder
eine erste Schicht, die aus einem porösen Material gebildet ist, das Collagen und ein Calciumphosphatmaterial beinhaltet; und eine zweite Schicht, die an die erste Schicht angefügt ist und aus einem Material gebildet ist, das ein Copräzipitat aus Collagen und einem Calciumphosphatmaterial beinhaltet; oder
eine erste Schicht, die aus einem porösen Material gebildet ist, das Collagen und ein Calciumphosphatmaterial beinhaltet; und eine zweite Schicht, die an die erste Schicht angefügt ist und aus einem Material gebildet ist, das ein dreifaches Copräzipitat aus Collagen, einem Glycosaminoglycan und einem Calciumphosphatmaterial beinhaltet; oder
eine erste Schicht, die aus einem porösen Material gebildet ist, das Collagen, ein Calciumphosphatmaterial und ein Glycosaminoglycan beinhaltet; und eine zweite Schicht, die an die erste Schicht angefügt ist und aus einem Material gebildet ist, das Collagen beinhaltet; oder
eine erste Schicht, die aus einem porösen Material gebildet ist, das Collagen, ein Calciumphosphatmaterial und ein Glycosaminoglycan beinhaltet; und eine zweite Schicht, die an die erste Schicht angefügt ist und aus einem Material gebildet ist, das ein Copräzipitat aus Collagen und einem Glycosaminoglycan beinhaltet; oder
eine erste Schicht, die aus einem porösen Material gebildet ist, das Collagen, ein Calciumphosphatmaterial und ein Glycosaminoglycan beinhaltet; und eine zweite Schicht, die an die erste Schicht angefügt ist und aus einem Material gebildet ist, das ein Copräzipitat aus Collagen und einem Calciumphosphatmaterial beinhaltet; oder
eine erste Schicht, die aus einem porösen Material gebildet ist, das Collagen, ein Calciumphosphatmaterial und ein Glycosaminoglycan beinhaltet; und eine zweite Schicht, die an die erste Schicht angefügt ist und aus einem Material gebildet ist, das ein dreifaches Copräzipitat aus Collagen, einem Glycosaminoglycan und einem Calciumphosphatmaterial beinhaltet.

16. Synthetisches Verbund-Biomaterial nach Anspruch 15, wobei zumindest ein Teil des Biomaterials aus einem porösen Copräzipitat gebildet ist, das Collagen und ein Calciumphsophatmaterial beinhaltet, wobei der Makroporen-Größenbereich (Porendurchmesser) bei 1 - 1200 Mikrometer oder bei 10 - 1000 Mikrometer oder bei 100 - 800 Mikrometer oder bei 200 - 600 Mikrometer liegt.

17. Synthetisches Verbund-Biomaterial nach Anspruch 15 oder 16, aufweisend: eine erste Schicht, die aus einem porösen Material gebildet ist, das ein dreifaches Copräzipitat aus Collagen, einem Calciumphosphatmaterial und einem Glycosaminoglycan beinhaltet; und eine zweite Schicht, die an die erste Schicht angefügt ist und aus einem Material gebildet ist, das ein Copräzipitat aus Collagen und einem Glycosaminoglycan beinhaltet.

18. Synthetisches Verbund-Biomaterial nach einem der Ansprüche 15 bis 17, eine oder mehrere weitere Schichten aufweisend, die an die erste und/oder zweite Schicht angefügt sind, wobei jede von den weiteren Schichten aus einem Material gebildet ist, das Collagen oder ein Copräzipitat aus Collagen und einem Glycosaminoglycan oder ein Copräzipitat aus Collagen und einem Calciumphosphatmaterial oder ein dreifaches Copräzipitat aus Collagen, einem Glycosaminoglycan und mindestens einem Calciumphosphatmaterial beinhaltet.

19. Synthetisches Verbund-Biomaterial nach einem der Ansprüche 15 bis 18, wobei die erste, die zweite und die mindestens eine weitere Schicht durch Flüssigphasen-Cosynthese als Einheit ausgebildet sind.

20. Synthetisches Verbund-Biomaterial nach einem der Ansprüche 15 bis 19, wobei aneinander angrenzende Schichten über eine Interdiffusionsschicht aneinander gefügt sind.

21. Synthetisches Verbund-Biomaterial nach einem der Ansprüche 15 bis 17, wobei die ersten und zweiten Schichten über eine Zwischenschicht aneinander gefügt sind.

22. Synthetisches Verbund-Biomaterial nach einem der Ansprüche 15 bis 21, wobei das Calciumphosphatmaterial ausgewählt ist aus einem oder mehreren von Brushit, Octacalciumphosphat und/oder Apatit.

23. Synthetisches Verbund-Biomaterial nach einem der Ansprüche 15 bis 22, wobei das Biomaterial Collagen und ein Glycosaminoglycan beinhaltet, und wobei das Collagen und das Glycosaminoglycan vernetzt sind.

24. Synthetisches Verbund-Biomaterial nach einem der Ansprüche 15 bis 23, wobei das Collagen in dem Material in einer Menge von 1 bis 99 Gew.-%, von 5 bis 90 Gew.-% oder von 15 bis 60 Gew.-% vorliegt.

25. Synthetisches Verbund-Biomaterial nach einem der Ansprüche 15 bis 24, wobei das Glycosaminoglycan in dem Material in einer Menge von 0,01 bis 20 Gew.-% oder von 1 bis 5,5 Gew.-% vorliegt.

26. Synthetisches Knochenmaterial, Knochenimplantat, Knochentransplantat, Knochenersatz, Knochenstützstruktur, Füllmaterial, Beschichtung oder Zement, ein synthetisches Verbund-Biomaterial nach einem der Ansprüche 15 bis 25 aufweisend.

27. In Schichten aufgebaute Knochenstützstruktur zur Verwendung im Tissue-Engineering, ein synthetisches Verbund-Biomaterial nach einem der Ansprüche 15 bis 25 aufweisend.

## Revendications

1. Un processus de préparation d'un biomatériau composite approprié à la réparation d'interfaces entre des tissus contenant du collagène et un matériau de phosphate de calcium, ou du collagène, un matériau de phosphate de calcium et un glycosaminoglycane, le processus comprenant :
(a) la fourniture d'une première composition de boue contenant un vecteur liquide, du collagène et un matériau de phosphate de calcium, et la fourniture d'une deuxième composition de boue contenant un vecteur liquide et du collagène, ou
la fourniture d'une première composition de boue contenant un vecteur liquide, du collagène et un matériau de phosphate de calcium, et la fourniture d'une deuxième composition de boue contenant un vecteur liquide, du collagène et un matériau de phosphate de calcium, ou
la fourniture d'une première composition de boue contenant un vecteur liquide, du collagène et un matériau de phosphate de calcium, et la fourniture d'une deuxième composition de boue contenant un vecteur liquide, du collagène, un matériau de phosphate de calcium et un glycosaminoglycane, ou
la fourniture d'une première composition de boue contenant un vecteur liquide, du collagène, un matériau de phosphate de calcium et un glycosaminoglycane, et la fourniture d'une deuxième composition de boue contenant un vecteur liquide et du collagène, ou
la fourniture d'une première composition de boue contenant un vecteur liquide, du collagène, un matériau de phosphate de calcium et un glycosaminoglycane, et la fourniture d'une deuxième composition de boue contenant un vecteur liquide, du collagène et un matériau de phosphate de calcium, ou
la fourniture d'une première composition de boue contenant un vecteur liquide, du collagène, un matériau de phosphate de calcium et un glycosaminoglycane, et la fourniture d'une deuxième composition de boue contenant un vecteur liquide, du collagène, un matériau de phosphate de calcium et un glycosaminoglycane,
(b) la fourniture d'un moule pour la boue,
(c) le dépôt des première et deuxième boues dans le moule,
(d) le refroidissement de la boue déposée dans le moule à une température à laquelle le vecteur liquide se transforme en une pluralité de particules ou cristaux solides,
(e) le retrait d'au moins une partie de la pluralité de particules ou cristaux solides par sublimation et/ou évaporation de façon à laisser un matériau composite poreux contenant du collagène, un matériau de phosphate de calcium, et éventuellement un glycosaminoglycane, et
(f) le retrait du matériau du moule.

2. Un processus selon la Revendication 1, où la première boue contient un coprécipité de collagène et du matériau de phosphate de calcium ou un coprécipité triple de collagène, d'un glycosaminoglycane et d'un matériau de phosphate de calcium, et la deuxième boue comprend un coprécipité de collagène et d'un glycosaminoglycane, un coprécipité de collagène et du matériau de phosphate de calcium ou un coprécipité triple de collagène, d'un glycosaminoglycane et d'un matériau de phosphate de calcium.

3. Un processus selon la Revendication 2, où la première boue et/ou la deuxième boue contient un coprécipité triple de collagène, du matériau de phosphate de calcium et d'un glycosaminoglycane.

4. Un processus selon l'une quelconque des Revendications 1 à 3, où le matériau de phosphate de calcium comprend de la brushite.

5. Un processus selon l'une quelconque des Revendications précédentes comprenant en outre :
la fourniture d'une autre composition de boue contenant un vecteur liquide et du collagène, ou la fourniture d'une autre composition de boue contenant un vecteur liquide, du collagène et un matériau de phosphate de calcium, ou
la fourniture d'une autre composition de boue contenant un vecteur liquide, du collagène, un matériau de phosphate de calcium et un glycosaminoglycane, et
avant ladite opération de refroidissement, le dépôt de ladite autre composition de boue dans le moule soit avant ou après que ladite première composition de boue ait été déposée.

6. Un processus selon l'une quelconque des Revendications précédentes, où les opérations (d) et (e) sont exécutées par une technique de lyophilisation.

7. Un processus selon l'une quelconque des Revendications précédentes, où le vecteur liquide contient de l'eau.

8. Un processus selon l'une quelconque des Revendications précédentes, où la température de la boue déposée dans le moule avant l'opération de refroidissement se situe dans la plage de 2 à 40°C ou de 4 à 37°C ou de 20 à 37°C.

9. Un processus selon l'une quelconque des Revendications précédentes, où l'opération de refroidissement de la boue déposée dans le moule est réalisée à une température dans la plage de -100 à 0°C ou de -80 à -10°C ou de -40 à -20°C.

10. Un processus selon l'une quelconque des Revendications précédentes, où l'opération de retrait d'au moins une partie des particules ou cristaux solides par sublimation est réalisée à une pression de 0 à 0,08 kPa ou de 0,0025 à 0,08 kPa ou de 0,0025 à 0,04 kPa.

11. Un processus selon l'une quelconque des Revendications précédentes, où l'opération de retrait d'au moins une partie des particules ou cristaux solides par sublimation est réalisée pendant jusqu'à 96 heures ou pendant de 12 à 72 heures ou pendant de 24 à 36 heures.

12. Un processus selon l'une quelconque des Revendications précédentes, où l'opération de retrait d'au moins une partie des particules ou cristaux solides par sublimation est réalisée à une température de -10 à 60°C ou de 0 à 40°C ou de 20 à 37°C ou de 25 à 37°C.

13. Un processus selon l'une quelconque des Revendications précédentes, comprenant en outre l'opération de réticulation du collagène et des un ou plusieurs glycosaminoglycanes dans le biomatériau composite poreux.

14. Un processus selon l'une quelconque des Revendications précédentes, comprenant en outre l'opération de conversion d'au moins une partie du matériau de phosphate de calcium dans le biomatériau composite poreux en une phase phosphate de calcium différente.

15. Un biomatériau composite synthétique approprié à la réparation d'interfaces entre des tissus comprenant :
une première couche formée d'un matériau poreux contenant du collagène et un matériau de phosphate de calcium à la condition que le matériau de phosphate de calcium ne soit pas hydroxylapatite, et une deuxième couche jointe à la première couche et formée d'un matériau contenant du collagène, ou
une première couche formée d'un matériau poreux contenant du collagène et un matériau de phosphate de calcium et une deuxième couche jointe à la première couche et formée d'un matériau contenant un coprécipité de collagène et d'un glycosaminoglycane, ou
une première couche formée d'un matériau poreux contenant du collagène et un matériau de phosphate de calcium et une deuxième couche jointe à la première couche et formée d'un matériau contenant un coprécipité de collagène et d'un matériau de phosphate de calcium, ou
une première couche formée d'un matériau poreux contenant du collagène et un matériau de phosphate de calcium et une deuxième couche jointe à la première couche et formée d'un matériau contenant un coprécipité triple de collagène, d'un glycosaminoglycane et d'un matériau de phosphate de calcium, ou
une première couche formée d'un matériau poreux contenant du collagène, un matériau de phosphate de calcium et un glycosaminoglycane et une deuxième couche jointe à la première couche et formée d'un matériau contenant du collagène, ou
une première couche formée d'un matériau poreux contenant du collagène, un matériau de phosphate de calcium et un glycosaminoglycane et une deuxième couche jointe à la première couche et formée d'un matériau contenant un coprécipité de collagène et d'un glycosaminoglycane, ou
une première couche formée d'un matériau poreux contenant du collagène, un matériau de phosphate de calcium et un glycosaminoglycane et une deuxième couche jointe à la première couche et formée d'un matériau contenant un coprécipité de collagène et d'un matériau de phosphate de calcium, ou
une première couche formée d'un matériau poreux contenant du collagène, un matériau de phosphate de calcium et un glycosaminoglycane et une deuxième couche jointe à la première couche et formée d'un matériau contenant un coprécipité triple de collagène, d'un glycosaminoglycane et d'un matériau de phosphate de calcium.

16. Un biomatériau composite synthétique selon la Revendication 15, où au moins une partie du biomatériau est formé à partir d'un coprécipité poreux contenant du collagène et un matériau de phosphate de calcium, où la plage de taille des macropores (diamètre des pores) est de 1 à 1200 microns ou de 10 à 1000 microns ou de 100 à 800 microns ou de 200 à 600 microns.

17. Un biomatériau composite synthétique selon la Revendication 15 ou 16, comprenant une première couche formée d'un matériau poreux comprenant un coprécipité triple de collagène, d'un matériau de phosphate de calcium et d'un glycosaminoglycane et une deuxième couche jointe à la première couche et formée d'un matériau contenant un coprécipité de collagène et d'un glycosaminoglycane.

18. Un biomatériau composite synthétique selon l'une quelconque des Revendications 15 à 17 comprenant une ou plusieurs autres couches jointes à la première et/ou la deuxième couche, chacune desdites autres couches étant formée d'un matériau contenant du collagène ou un coprécipité de collagène et d'un glycosaminoglycane ou un coprécipité de collagène et d'un matériau de phosphate de calcium ou un coprécipité triple de collagène, d'un glycosaminoglycane et d'au moins un matériau de phosphate de calcium.

19. Un biomatériau composite synthétique selon l'une quelconque des Revendications 15 à 18, où les première, deuxième et lesdites une ou plusieurs autres couches sont formées d'un seul tenant par co-synthèse en phase liquide.

20. Un biomatériau composite synthétique selon l'une quelconque des Revendications 15 à 19, où des couches adjacentes sont jointes les unes aux autres par l'intermédiaire d'une couche de diffusion intermédiaire.

21. Un biomatériau composite synthétique selon l'une quelconque des Revendications 15 à 17, où les première et deuxième couches sont jointes les unes aux autres par l'intermédiaire d'une couche intermédiaire.

22. Un biomatériau composite synthétique selon l'une quelconque des Revendications 15 à 21, où le matériau de phosphate de calcium est sélectionné parmi un ou plusieurs des matériaux suivants : brushite, phosphate octacalcique et/ou apatite.

23. Un biomatériau composite synthétique selon l'une quelconque des Revendications 15 à 22, où le biomatériau contient du collagène et un glycosaminoglycane, et où le collagène et le glycosaminoglycane sont réticulés.

24. Un biomatériau composite synthétique selon l'une quelconque des Revendications 15 à 23, où le collagène est présent dans le matériau dans une quantité de 1 à 99% en poids, de 5 à 90% en poids ou de 15 à 60% en poids.

25. Un biomatériau composite synthétique selon l'une quelconque des Revendications 15 à 24, où le glycosaminoglycane est présent dans le matériau dans une quantité de 0,01 à 20% en poids ou de 1 à 5,5% en poids.

26. Un matériau osseux synthétique, un implant osseux, un greffon osseux, un substitut osseux, un échafaudage osseux, une charge, un revêtement ou un ciment contenant un biomatériau composite synthétique tel que défini dans l'une quelconque des Revendications 15 à 25.

27. Un échafaudage osseux en couches pour une utilisation en ingénierie tissulaire comprenant un biomatériau composite synthétique tel que défini dans l'une quelconque des Revendications 15 à 25.
